# EUROPÄISCHE PATENTSCHRIFT

(11) **EP 1 339 421 B1**
(45) Veröffentlichungstag und Bekanntmachung des Hinweises auf die Patenterteilung: **16.03.2005**
(21) Anmeldenummer: 01994746.4
(22) Anmeldetag: 28.11.2001
(51) Int. Cl.: A61K 35/78, A61K 7/48, A61K 7/06, A61P 17/00

(54) **KOSMETISCHE UND/ODER DERMOPHARMAZEUTISCHE ZUBEREITUNGEN ENTHALTEND NATIVE PROTEINE AUS DER PFLANZE ARGANIA SPINOSA**
COSMETIC AND/OR DERMOPHARMACEUTICAL PREPARATIONS CONTAINING NATIVE PROTEINS FROM THE PLANT ARGANIA SPINOSA
PREPARATIONS COSMETIQUES OU DERMOPHARMACEUTIQUES CONTENANT DES PROTEINES NATIVES DE LA PLANTE ARGANIA SPINOSA

(30) Priorität: 06.12.2000 EP 00440318
(43) Veröffentlichungstag der Anmeldung: 03.09.2003
(73) Patentinhaber: Cognis France S.A., 31360 Saint-Martory (FR)
(72) Erfinder: PAULY, Gilles, F-54000 Nancy (FR); HENRY, Florence, F-54600 Villers-les-Nancy (FR); MOSER, Philippe, F-54270 Essey les Nancy (FR); CHARROUF, Zoubida, B.P. 1014 Rabat R.P. (MA)
(74) Vertreter: Fabry, Bernd, Dr.
(86) Internationale Anmeldenummer: PCT/EP2001/013886
(87) Internationale Veröffentlichungsnummer: WO 2002/045729

(56) Entgegenhaltungen:
- FR-A- 2 500 306
- FR-A- 2 553 788
- FR-A- 2 724 663
- FR-A- 2 756 183
- ALAOUI K. ET AL: "Activité analgésique et anti-inflammatoire des saponines d'Argania spinosa" ANNALES PHARMACEUTIQUES FRANCAISES ISSN: 0003-4509 CODEN: APFRAD, Bd. 56, Nr. 5, 1998, Seiten 220-228, XP000995497 Laboratoire de Pharmacologie et Toxicologie, Faculte de Medecine et de Pharmacie, BP 6203, Rabat, Instituts, Agdal, Rabat, Morocco;Laboratoire de Chimie organique, Faculte de Pharmacie de Limoges, France; Departement de Biologie, Faculte des Sciences de Casa I, Morocco; Laboratoire de Pharmacologie,
- BERRADA Y. ET AL: "Mise en évidence expérimentale des effets antihypertenseurs et hypocholestérolémiants de l'huile d'Argan, Argania sideroxylon" THERAPIE, Bd. 55, Nr. 3, 2000, Seiten 375-378, XP000939171 Departement de pharmacologie, faculte de medecine et de pharmacie de Rabat, Morocco
- CHAROUF Z. ET AL: "Triterpènes et sterols extraits de la poulpe d'Argania spinosa (L.), Sapotaceae" PLANTES MEDICINALES ET PHYTOTHERAPIE ISSN: 0032-0994 CODEN: PLMPA9, Bd. 25, Nr. 2-3, 1991, Seiten 112-117, XP000619575 Fac. sci., lab. chmie plantes, 1014 Rabat, Morocco;Serv. commun, USTL Lille, 59650 Villeneuve d'Asq, France
- ALAOUI B F (REPRINT) ET AL: "Conservation study of the argan oil by thermogravimetry" ASIAN JOURNAL OF CHEMISTRY, ISSN: 0970-7077., Bd. 13, Nr. 1, 2001, Seiten 144-150, XP001062010 Univ Mohammed V, Fac Sci, Dept Chem, Lab React Solid Gas Syst, Rabat, Morocco
- BELLAKHDAR J ET AL: "REPERTORY OF STANDARD HERBAL DRUGS IN THE MOROCCAN PHARMACOPOEA" J ETHNOPHARMACOL, Bd. 35, Nr. 2, 1991, Seiten 123-143, XP001068322 PHARM CHERCHEUR, RABAT MOROCCO
- SCHAR M P: "ARGAN OIL" EURO COSMET, Bd. 5, 1999, Seiten 45-47, XP001062004
- PHYTOCHEMISTRY Bd. 31, Nr. 6, 1992, Seiten 2079 - 2086

## Beschreibung

### Gebiet der Erfindung

Die Erfindung befindet sich auf dem Gebiet der Pflegestoffe und betrifft Zubereitungen enthaltend native Proteine aus der Pflanze Argania spinosa sowie Verwendung von nativen Proteinen aus der Pflanze Argania spinosa als neue Haut - und Haarpflegemittel.

### Stand der Technik

Kosmetische Zubereitungen stehen dem Verbraucher heute in einer Vielzahl von Kombinationen zur Verfügung. Dabei wird nicht nur erwartet, dass diese Kosmetika einen bestimmten pflegenden Effekt zeigen oder einen bestimmten Mangel beheben, sondern immer häufiger wird nach Produkten verlangt, die mehrere Eigenschaften gleichzeitig aufweisen und somit ein verbessertes Leistungsspektrum zeigen. Von besonderem Interesse sind Stoffe, die sowohl Wirkstoffe darstellen, die für Haut und/oder Haare beispielsweise pflegende, vor Alterserscheinungen schützende, revitalisierende Eigenschaften vermitteln als auch gleichzeitig die technischen Eigenschaften des kosmetischen Produktes, wie Lagerstabilität, Lichtstabilität und Formulierbarkeit positiv beeinflussen oder zumindest nicht verschlechtern. Hierbei sind zusätzlich eine gute Hautverträglichkeit und besonders der Einsatz natürlicher Produkte beim Kunden gefragt Daneben ist es wünschenswert, durch Kombination bereits bekannter Wirkstoffe, oder durch auffinden neuer Einsatzgebiete bereits bekannter Substanzklassen deutlich bessere Produkte zu erhalten. Ein Nachteil besteht hier allerdings häufig darin, das eine Kombination von Wirkstoffen erst dann erhalten wird, wenn unterschiedliche Pflanzenextrakte gleichzeitig in unterschiedlichen Mengenverhältnissen verwendet werden.

Extrakte von Pflanzen und deren Inhaltstoffe finden immer häufiger Einsatz in der Kosmetik und Pharmazie. Pflanzenextrakte werden seit vielen Jahren in den unterschiedlichsten Kulturen für medizinische aber auch bereits für kosmetische Zwecke genutzt. Oftmals waren für diese Pflanzenextrakte nur ganz bestimmte einzelne Wirkungen bekannt und das Einsatzgebiet sehr eingeschränkt.

### Beschreibung der Erfindung

FR-A 2 756183 offenbart die kosmetischen und therapeutischen Effekte einer Peptidfraktion aus den Samenkernen von Argania spinosa. Diese Peptidfraktion weist ein Molekulargewicht von <20,000 Dalton auf und wird durch enzymatische Hydrolyse der Proteine gewonnen.

Die Publikation Alaoui K. et al.; Activite analgesique et anti-inflammatoire des saponines d'Argania spinosa, Annales pharmaceutiques francaises, Bd. 56, Nr. 5, 1998, Seiten 220-228, offenbart die schmerzlindemden und entzündungshemmenden Aktivitäten eines Extraktes aus dem Rückstand der Extraktion zur Arganölgewinnung, die Extraktion erfolgt durch Anwendung von Ethanol. Demgemäß handelt es sich um einen Saponin-Extrakt und nicht um einen Protein-Extrakt der Pflanze Argania spinosa.

Die Aufgabe der vorliegenden Patentanmeldung hat darin bestanden, kosmetische und/oder dermopharmazeutische Zubereitungen zur Verfügung zu stellen, die einen Einsatz in der Kosmetik oder auch der Pharmazie ermöglichen und neben pflegenden Eigenschaften vor allem verbesserte feuchtigkeitsregulierende und schützende Eigenschaften für menschliche Haut und/oder Haare besitzen und gleichzeitig vorbeugende und heilende Wirkung bei Alterserscheinungen der Haut zeigen, reaktivierend und revitalisierend wirken können und als Schutz gegen UV-Strahlung einsetzbar sind.

Eine weitere Aufgabe der vorliegenden Patentanmeldung hat darin bestanden, Zubereitungen zur Verfügung zu stellen, die Wirkstoffe aus nachwachsenden Rohstoffen enthalten und gleichzeitig vielseitig als Pflegemittel in der Kosmetik sowohl in der Hautkosmetik, als auch in der Haarpflege einsetzbar sind.

Gegenstand der Erfindung sind Zubereitungen, die native Proteine aus der Pflanze Argania spinosa enthalten als Pflegemittel für Haut und Haare.

Überraschenderweise wurde gefunden, dass durch den Einsatz von nativen Proteinen aus der Pflanze Argania spinosa Produkte erhalten werden, die gleichzeitig gute pflegende und schützende Eigenschaften für Haut und Haar aufweisen, sowie eine hohe Hautverträglichkeit besitzen. Die so erhaltenen Mittel zeichnen sich durch besonders gute Effekte in der Hautkosmetik aus. Sie zeigen neben feuchtigkeitsregulierende und schützende Effekte auch eine vorbeugende und heilende Wirkung bei Alterserscheinungen der Haut und eine revitalisierenden und reaktivierende Aktivität auf Haut und Haare.

Diese mehrfachen Einsatzgebiete der erfindungsgemäßen Mittel aus dem nachwachsendem Rohstoff der Pflanze Argania spinosa macht es für den Markt und für den Verbraucher sehr attraktiv. Die komplexe Aufgabe der Erfindung konnte somit durch den Einsatz von nativen Proteinen aus der Pflanze Argania spinosa gelöst werden.

Der Begriff Zubereitungen wird im Rahmen der Erfindung synonym mit dem Begriff Mittel oder Pflegemittel verwendet.

Unter dem Begriff Pflanze im Sinne der vorliegenden Anmeldung sind sowohl ganze Pflanzen als auch Pflanzenteile (Samenkerne, Blätter, Wurzeln, Blüten) sowie deren Gemische zu verstehen.

### Argania spinosa

Die erfindungsgemäß einzusetzenden Extrakte werden aus Pflanzen der Familie der Sapotaceae, speziell aus Argania spinosa gewonnen. Bei dieser Pflanze handelt es sich um einen an den Ölbaum erinnernden Baum, der überwiegend in Marokko an der Westseite des Atlasgebirges zu finden ist. Er bildet an seinen knorrigen Ästen und bedomten Zweigen Beeren von der Größe und Gestalt der Oliven mit ein bis zwei Samenkernen. Das nussartig schmeckende Öl aus den Samenkernen dient unter anderem als Speiseöl.

### Proteine

Unter **Proteinen** sind im Sinne der Erfindung solche zu verstehen, die sich aus der Pflanze Argania spinosa isolieren lassen. Proteine (Eiweiß) bilden die aktiven Enzyme in allen Zellkernen und stellen die Reserve zur Bildung neuer Enzyme. Aus dem Grund sind sie ein wichtiger Bestandteil der Pflanzen und finden sich daher in allen Pflanzenteilen. Besonders bevorzugt ist die Extraktion der Samenkerne, insbesondere der entfetteten Samenkerne. Dementsprechend ist eine besondere Ausführungsform der Erfindung Zubereitungen, die native Proteine enthalten, die erhalten werden aus einem Extrakt der Samenkerne, insbesondere der entfetteten Samenkerne von Argania spinosa.

Unter der bevorzugten Extraktion der entfetteten Samenkerne ist im Sinne der Erfindung zu verstehen, dass bevorzugt der Rückstand - eine Art Kuchen - aus der Extraktion zur Ölgewinnung aus den Samenkernen von Argania spinosa extrahiert wird. Dieser bevorzugt zu extrahierende Rückstand aus der Extraktion zur Ölgewinnung enthält 3 bis 10 Gew.-% restliches Öl. Aus diesem Rückstand werden die erfindungsgemäßen Proteine vom noch verbliebenen Öl möglichst vollständig getrennt. Neben Proteinen können noch weitere, natürlich in den Pflanzen Argania spinosa vorkommende Substanzen mit extrahiert werden, die unter den gleichen Bedingungen extrahierbar sind.

In einer weiteren Ausführungsform der Erfindung enthalten die erfindungsgemäßen Zubereitungen native Proteine, die durch wässrige Extraktion bei einem pH-Wert geringer oder gleich 12, bevorzugt zwischen 3,5 und 6,5, insbesondere entweder zwischen 5,5 und 6,5 oder zwischen 3,5 und 5,5 und gegebenenfalls einer anschließenden Trocknung, beispielsweise einer Sprüh- oder Gefriertrocknung erhalten werden. Der gewählte pH-Wert Bereich ist abhängig von der zu isolierenden Proteinfraktion.

Die nativen Proteine, die sich aus der Pflanze Argania spinosa, insbesondere aus den Samenkernen der Pflanze extrahieren lassen, können Molekulargewichte zwischen 10.000 Da und größer als 500.000 Da besitzen. Bevorzugt können sie eingeteilt werden in folgende Gruppen von Molekulargewichtsbereichen. Extrahiert werden können native Proteine mit einem Molekulargewicht größer als 500.000 Da, native Proteine mit Molekulargewicht im Bereich von 170.000 bis 250.000 Da und native Proteine mit Molekulargewicht im Bereich von 10.000 bis 18.000 Da.

Demnach betreffen weitere Ausführungsformen der Erfindung zum einen Zubereitungen, die native Proteine enthalten, deren Molekulargewicht größer als 500.000 Da beträgt, Zubereitungen, die native Proteine enthalten, deren Molekulargewicht im Bereich von 170.000 Da bis 250.000 Da, bevorzugt im Bereich von 170.000 Da bis 210.000 Da liegt und Zubereitungen, die native Proteine enthalten, deren Molekulargewicht im Bereich von 10.000 bis 18.000 bevorzugt im Bereich von 13.000 bis 16.000 liegt.

Der Anteil an nativen Proteinen, berechnet auf das Trockengewicht des Extraktes liegt zwischen 20 und 60 Gew.-%, insbesondere 35 bis 55 Gew.-%. Demnach ist eine weitere besondere Ausführungsform der Erfindung Zubereitungen, die native Proteine in Form eines Extraktes mit einem Aktivsubstanzgehalt im Bereich von 20 bis 85 Gew.-%, insbesondere 35 bis 55 Gew.-% oder 60 bis 85 Gew.-%- berechnet auf Trockengewicht, je nach Extraktionsmethode - enthalten.

### Extraktion

Die Herstellung der erfindungsgemäß einzusetzenden Extrakte erfolgt durch übliche Methoden der Extraktion von Pflanzen bzw. Pflanzenteilen. Bezüglich der geeigneten herkömmlichen Extraktionsverfahren wie der Mazeration, der Remazeration, der Digestion, der Bewegungsmazeration, der Wirbelextraktion, Ultraschallextraktion, der Gegenstromextraktion, der Perkolatian, der Reperkofation, der Evakolation (Extraktion unter vermindertem Druck), der Diakolation und Festflüssig-Extraktion unter kontinuierlichem Rückfluß, die in einem Soxhlet-Extraktor durchgeführt wird, die dem Fachmann geläufig und im Prinzip alle anwendbar sind, sei beispielhaft auf **Hagers Handbuch der Pharmazeutischen Praxis,** (5. Auflage, Bd. 2, S. 1026-1030, Springer Verlag, Berlin-Heidelberg-New-York 1991) verwiesen. Als Ausgangsmaterial können frische oder getrocknete Pflanzen oder Pflanzenteile eingesetzt werden, üblicherweise wird jedoch von Pflanzen und/oder Pflanzenteilen ausgegangen, die vor der Extraktion mechanisch zerkleinert werden können. Hierbei eignen sich alle dem Fachmann bekannten Zerkleinerungsmethoden, als Beispiel sei die Zerkleinerung mit einem Klingen enthaltenen Gerät genannt. Bevorzugt wird der Rückstand aus der Ölgewinnung aus den Samenkernen der Pflanze extrahiert.

Als Lösungsmittel für die Durchführung der Extraktionen wird als Lösungsmittel, Wasser verwendet. Die Extraktion erfolgt in der Regel bei 20 bis 100 °C, bevorzugt bei 80 bis 100°C, insbesondere bei Siedetemperatur der Lösungsmittel oder Lösungsmittelgemische. In einer möglichen Ausführungsform erfolgt die Extraktion unter Inertgasatmosphäre zur Vermeidung der Oxidation der Inhaltsstoffe des Extraktes. Die Extraktionszeiten werden vom Fachmann in Abhängigkeit vom Ausgangsmaterial, dem Extraktionsverfahren, der Extraktionstemperatur, vom Verhältnis Lösungsmittel zu Rohstoff u.a. eingestellt. Nach der Extraktion können die erhaltenen Rohextrakte gegebenenfalls weiteren üblichen Schritten, wie beispielsweise Aufreinigung, Konzentration und/oder Entfärbung unterzogen werden. Falls wünschenswert, können die so hergestellten Extrakte beispielsweise einer selektiven Abtrennung einzelner unerwünschter Inhaltsstoffe, unterzogen werden. Die Extraktion kann bis zu jedem gewünschten Extraktionsgrad erfolgen, wird aber gewöhnlich bis zur Erschöpfung durchgeführt.

Die vorliegende Erfindung umfasst die Erkenntnis, dass die Extraktionsbedingungen sowie die Ausbeuten der Endextrakte je nach gewünschtem Einsatzgebiet gewählt werden können.

Die Einsatzmenge der Pflanzenextrakte in den genannten Zubereitungen richtet sich nach der Konzentration der einzelnen Inhaltstoffe und nach der Art der Anwendungen der Extrakte. Die Gesamtmenge des die nativen Proteine enthaltenden Pflanzenextraktes, der in den erfindungsgemäßen Zubereitungen enthalten ist, beträgt in der Regel 0,01 bis 25 Gew.-%, vorzugsweise 0,03 bis 5 Gew.-%, insbesondere 0,03 bis 0,6 Gew.-% bezogen auf die Zubereitungen, mit der Maßgabe, dass sich die Mengenangaben mit Wasser und gegebenenfalls weiteren Hilfs- und Zusatzstoffen zu 100 Gew.-% addieren.

Der Gesamtanteil der Hilfs- und Zusatzstoffe kann 1 bis 50, vorzugsweise 5 bis 40 Gew.-% - bezogen auf die Endzubereitung der kosmetischen und/oder dermopharmazeutischen Zubereitungen - betragen.

Die Herstellung der Zubereitungen kann durch übliche Kalt - oder Heißprozesse erfolgen; vorzugsweise arbeitet man nach der Phaseninversionstemperatur-Methode.

Aktivsubstanz im Sinne der Erfindung bezieht sich auf den Anteil an Substanzen sowie Hilfs- und Zusatzstoffen, die in dem Mittel enthaltend sind, mit Ausnahme des zusätzlich hinzugefügten Wassers.

Ein weiterer Gegenstand der Erfindung ist die Verwendung von nativen Proteinen aus Extrakten der Pflanze Argania spinosa als Pflegemittel für die Haut und/oder die Haare. Diese Art der Verwendung umfasst sowohl Mittel mit kosmetischer als auch mit dermopharmazeutischer Wirkung.

### Pflegemittel:

Als Pflegemittel im Sinne der Erfindung sind Pflegemittel für Haut und Haar zu verstehen. Diese Pflegemittel schließen unter anderem reinigende und aufbauende Wirkung für Haut und Haare ein.

Die Applikation kann sowohl topisch als auch oral in Form von Tabletten, Dragees, Kapseln, Säfte, Lösungen und Granulate erfolgen.

Die erfindungsgemäßen Zubereitungen zeigen darüber hinaus eine hervorragende hautpflegende Wirkung bei gleichzeitig hoher Hautverträglichkeit. Außerdem zeigen sie eine gute Stabilität, insbesondere gegenüber oxidativer Zersetzung der Produkte. Die Zubereitungen weisen eine Vielzahl von kosmetischen und dermopharmazeutischen Wirkungen auf. Weitere Gegenstände der Erfindung betreffen daher die Verwendung von nativen Proteinen aus Extrakten der Pflanze Argania spinosa
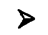 als Pflegemittel für Haut und/oder Haare;
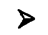 als feuchtigkeitsregulierendes Feuchthaltemittel;
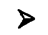 als Mittel zur Stärkung der Hautbarrierefunktionen
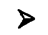 als hautstraffendes und hautstabilisierendes Mittel;
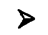 als Sonnenschutzmittel, insbesondere gegen UVA-Strahlung und/oder gegen UVB-Strahlung;
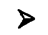 als Mittel gegen die Hautalterung;
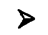 als revitalisierendes und restrukturierendes Mittel für Haut und/oder Haare;
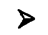 als Wirkstoffe zur Herstellung eines Mittels zur Steigerung der G6PDH-Aktivität im Stoffwechsel;

Die nativen Proteine aus Extrakten der Pflanze Argania spinosa wirken im Sinne der Erfindung als feuchtigkeitsregulierende Feuchthaltemittel. Im Sinne der Erfindung sind darunter Hautpflegemittel zu verstehen, die der Feuchtigkeitsregulierung der Haut dienen. Dieses entspricht im Sinne der Erfindung der Definition eines Moisturizer. Es sind Stoffe oder Stoffgemische, die kosmetischen und/oder dermopharmazeutischen Zubereitungen die Eigenschaft verleihen, nach dem Auftragen und Verteilen auf der Hautoberfläche, die Feuchtigkeitsabgabe des Stratum corneum (Hornschicht) zu reduzieren.

Die erfindungsgemäßen Feuchthaltemittel enthalten native Proteine aus Extrakten der Pflanze Argania spinosa. Als weitere Feuchthaltemittel können in Kombination mit den native Proteinen aus dem Pflanzenextrakt beispielhaft weitere Feuchthaltemittel enthalten sein, wie:
- Polyglycerinfettsäureester auf Basis von Fettsäuren mit 12-18 C-Atomen, zB. Tetraglycerylmonooleat, Triglyceryldiisostearat;
- Pyroglutaminsäure oder L-Argininpyroglutamat, L-Lysinpyroglutamat;
- Mischungen von Aminosäuren wie z.B. L-Alanin, L-Arginin, L-Serin, L-Threonin;
- Propylen Glycol
- Acetamid
- Polysaccharide oder Hyaloronsäure
- Ricinusölether und Sorbitanester wie in der **JP60149511** (Lion Corp) beschrieben

### Sonnenschutzmittel bzw. UV-Lichtschutzfaktoren

Die nativen Proteine aus Extrakten der Pflanze Argania spinosa wirken im Sinne der Erfindung als Sonnenschutzmittel.

Als Sonnenschutzmittel bzw. UV-Lichtschutzfaktoren im Sinne der Erfindung werden Lichtschutzmittel bezeichnet, die für den Schutz der menschlichen Haut gegenüber schädigenden Einflüssen der direkten und indirekten Strahlung der Sonne nützlich sind. Die für die Hautbräunung verantwortliche Ultraviolettstrahlung der Sonne unterteilt man in die Abschnitte UV-C (Wellenlängen 200-280 nm), UV-B (280-315 nm) u. UV-A (315-400 nm).

Die Pigmentierung normaler Haut unter dem Einfluss der Sonnenstrahlung, d. h. die Bildung von Melaninen, wird durch UV-B u. UV-A unterschiedlich bewirkt. Bestrahlung mit UV-A-Sarahlen ("langwelligem UV") hat die Dunkelung der in der Epidermis bereits vorhandenen Melanin-Körper zur Folge, ohne dass schädigende Einflüsse zu erkennen sind. Anders bei dem sog. "kurzwelligen UV" (UV-B). Dieses bewirkt die Entstehung von sog. Spätpigment durch Neubildung von Melanin-Körnern. Ehe jedoch das (schützende) Pigment gebildet ist, unterliegt die Haut der Einwirkung der ungefilterten Strahlung, die - je nach Expositionsdauer - zur Bildung von Hautrötungen (Erythemen), Hautentzündungen (Sonnenbrand) u. gar Brandblasen führen kann.

Als UV-Absorber oder Uchtfilter, die also die UV-Strahlung in unschädliche Wärme umwandeln, werden native Proteine aus Extrakten aus der Pflanze Argania spinosa eingesetzt, diese können zusätzlich in Kombination mit weiteren Sonnenschutzmitteln bzw. UV-Lichtschutzfaktoren vorliegen.

Diese weiteren UV- Lichtschutzfaktoren sind beispielsweise bei Raumtemperatur flüssig oder kristallin vorliegende organische Substanzen (Lichtschutzfilter), die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche Substanzen sind z.B. zu nennen:
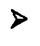 3-Benzylidencampher bzw. 3-Benzylidennorcampher und dessen Derivate, z.B. 3-(4-Methylbenzyliden)campher wie in der **EP 0693471 B1** beschrieben;
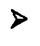 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester,
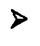 Ester der Zimtsäure, vorzugsweise 4-Methoxyzimtsäure-2-ethylhexylester, 4-Methoxyzimtsäurepropylester, 4-Methoxyzimtsäureisoamylester 2-Cyano-3,3-phenylzimtsäure-2-ethylhexylester (Octocrylene);
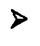 Ester der Salicylsäure, vorzugsweise Salicylsäure-2-ethylhexylester, Salicylsäure-4-isopropylbenzylester, Salicylsäurehomomenthytester,
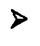 Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
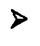 Ester der Benzalmalonsäure, vorzugsweise 4-Methoxybenzmalonsäuredi-2-ethylhexylester;
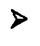 Triazinderivate, wie z.B. 2,4,6-Trianilino-(p-carbo-2'-ethyl-1'-hexyloxy)-1,3,5-triazin und Octyl Triazon, wie in der EP 0818450 A1 beschrieben oder Dioctyl Butamido Triazone (Uvasorb® HEB);
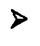 Propan-1,3-dione, wie z.B. 1-(4-tert.Butylphenyl)-3-(4'methoxyphenyl)propan-1,3-dion;
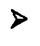 Ketotricyclo(5.2.1.0)decan-Derivate, wie in der **EP 0694521 B1** beschrieben.

Als wasserlösliche Substanzen kommen in Frage:
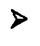 2-Phenylbenzimidazol-5-sulfonsäure und deren Alkali-, Erdalkali-, Ammonium-, Alkylammonium-, Alkanolammonium- und Glucammoniumsalze;
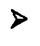 Sulfonsäurederivate von Benzophenonen, vorzugsweise 2-Hydroxy-4-methoxybenzophenon-5-sulfonsäure und ihre Salze;
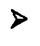 Sulfonsäurederivate des 3-Benzylidencamphers, wie z.B. 4-(2-Oxo-3-bornylidenmethyl)benzolsulfonsäure und 2-Methyl-5-(2-oxo-3-bomyliden)sulfonsäure und deren Salze.

Als typische UVA-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol 1789), 1-Phenyl-3-(4'-isopropylphenyl)-propan-1,3-dion sowie Enaminverbindungen, wie beispielsweise beschrieben in der **DE 19712033 A1** (BASF). Die UV-A und UV-B-Filter können selbstverständlich auch in Mischungen eingesetzt werden. Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000 (Merck). Als hydrophobe Coatingmittel kommen dabei vor allem Silicone und dabei speziell Trialkoxyoctylsilane oder Dimethicone in Frage. In Sonnenschutzmitteln werden bevorzugt sogenannte Mikro- oder Nanopigmente eingesetzt. Vorzugsweise wird mikronisiertes Zinkoxid verwendet. Weitere geeignete UV-Lichtschutzfilter sind der Übersicht von P.Finkel in **SÖFW.Journal 122, 543 (1996)** sowie **Parfümerie und Kosmetik 3 (1999), Seite 11ff** zu entnehmen.

Die nativen Proteine aus Extrakte der Pflanze Argania spinosa wirken im Sinne der Erfindung gegen die Schädigung von Fibroblasten und/oder Keratinocyten durch UVA-Strahlung und/oder UVB-Strahlung.

UVA-Strahlen dringen bis in die Dermis ein, wo sie zu Oxidationsstreß führen, was durch eine Lipoperoxidation der Zytoplasmamembranen nachgewiesen wird. Die Lipoperoxide werden zu Malonaldialdehyd (MDA) abgebaut, der viele biologische Moleküle wie Proteine und Nukleinbasen vernetzen wird (Enzymhemmung bzw. Mutagenese). Die erfindungsgemäßen Extrakte der Pflanze Argania spinosa reduzieren signifikant den Grad an MDA in humanen Fibroblasten, welcher durch UVA-Strahlen induziert wird und zeigen damit eine hohe Kapazität schädliche Effekte eines oxidativen Stresses auf der Haut zu reduzieren.

UVB-Strahlen lösen durch Aktivierung eines Enzyms, nämlich Phospholipase A2 oder PLA2 eine Entzündung aus. Diese Entzündung (Erythem, Ödem) wird durch die Entfernung von Arachidonsäure aus den Phospholipiden der Plasmamembran durch die Phospholipase ausgelöst. Arachidonsäure ist die Vorstufe der Prostaglandine, die eine Entzündung und eine Zellmembranschädigung verursachen; die Prostaglandine E2 (= PGE2) werden durch die Cyclooxygenase gebildet Der Grad der Freisetzung des Cytoplasaenzyms LDH (Lactat Dehydrogenase) in humanen Keratinocyten dient als Marker für eine Zellschädigung.

Die erfindungsgemäßen nativen Proteine aus Extrakten der Pflanze Argania spinosa reduzieren den Effekt von UVB-Strahlung auf die Anzahl an Keratinocyten und auf den Gehalt an freigesetzte LDH. Die Extrakte zeigen demnach die Fähigkeit, die durch UVB-Strahlung hervorgerufene Schädigung an Zellmembranen zu reduzieren.

Die nativen Proteine aus Extrakten der Pflanze Argania spinosa wirken im Sinne der Erfindung gegen Hautalterungen, insbesondere gegen jede Art der Fältchen- und Faltenbildung. Eine andere Bezeichnung für diese Art der Pflegemittel ist auch anti-ageing Mittel. Die Verwendungen schließen eine Verlangsamung von Altersprozessen der Haut mit ein. Die Alterserscheinungen können die unterschiedlichsten Ursachen aufweisen. Insbesondere können diese Alterserscheinungen auf Grund von Apoptose, durch UV-Strahlung oder durch die Zerstörung der hauteigenen Proteine wie beispielsweise Collagen oder Elastan induzierten Schädigungen der Haut verursacht sein.

Die nativen Proteine aus Extrakten der Pflanze Argania spinosa wirken im Sinne der Erfindung als schützende und aufbauende Pflegemittel mit revitalisierenden und reaktivierenden Aktivitäten für die Haut und/oder Haare. Diese Art der Verwendung dieser Pflegemittel, wirkt positiv beispielsweise gegen den negativen Einfluss der Umweltverschmutzung auf Haut und/oder Haare, indem sie die natürlichen Funktionen von Haut und/oder Haare reaktivieren und die Haut und/oder Haare widerstandsfähiger machen. Die revitalisierende und reaktivierende Aktivität von nativen Proteinen aus Extrakten der Pflanze Argania spinosa wirkt der Apoptose entgegen. Die erfindungsgemäße Lehre schließt die Erkenntnis mit ein, dass die nativen Proteine aus Extrakten der Pflanze Argania spinosa als hautstraffendes und hautstabilisierendes Mittel wirken.

Im Sinne der Erfindung versteht man unter Apoptose den gezielten Zelltod bestimmter unerwünschter oder geschädigter Zellen. Es handelt sich um einen aktiven Prozess der Zellen (Selbstmord auf Befehl). Apoptose wird durch einen oxidativen Stress (UV-Strahlung, Entzündung), durch einen Mangel an Wachstumsfaktoren oder durch giftige Stoffe (Schmutzstoffe, genotoxische Stoffe usw.) eingeleitet Bei der Hautalterung z. B. kann es durch einen Mangel an Wachstumsfaktoren in der Haut zu einer induzierten Apoptose der Hautzellen kommen. Bei den durch Apoptose betroffenen Zellen wird durch das spezifische Enzym Endonuclease die nukleare DNA abgebaut und die DNA-Fragmente in das Cytoplasma geschleust Als Wachstumsfaktoren im Sinne der Erfindung sind prinzipiell alle körpereigenen oder von außen zugeführten zu verstehen, die das Wachstum von Haut- und Haarzellen stimulieren. Dazu zählen beispielsweise Hormone und chemische Mediatoren oder Signalmoleküle. Es handelt sich zum Beispiel um Polypeptid-Wachstumsfaktoren oder Glykoprotein-Wachstumsfaktoren. Hier sei der Epidermale Wachstumsfaktor (EGF) genannt, der aus 53 Aminosäuren besteht und damit einen Polypeptid Wachstumsfaktor darstellt oder das Fibrillin, welches zu den Glykoproteinen gehört. Weitere Wachstumsfaktoren sind beispielsweise Urogastron, Laminin, Follistatin und Heregelin.

Die erfindungsgemäßen nativen Proteine aus Extrakten der Pflanze Argania spinosa können als Wirkstoffe zur Herstellung der Steigerung der G6PDH Aktivität im Stoffwechsel eingesetzt werden, da sie nachweislich die enzymatische Aktivität dieses für den Stoffwechsel wichtigen Enzymes erhöhen.

Glucose-6-phosphat Dehydrogenase (G6PDH) katalysiert den ersten Schritt des oxidativen Zweiges des Pentosephosphat-Weges. In diesem ersten Schritt wird Glucose-6-phosphat zu 6-Phosphono-δ-lacton unter Einwirkung von NADP. Dieses Coenzym wird bei dieser Oxidation zu NADPH2 reduziert. Die reduzierte Form dieses Coenzyms kann viele enzymatische Reaktionen wie beispielsweise Recycling von Glutathion oder Upidsynthese katalysieren. Des weiteren produziert der Pentosephosphat-Weg eine essentielle Komponenten für den Aufbau der DNA, die Desoxyribose. Reduziertes Glutathion schütz viele Enzyme mit der "SH"-Gruppe und fördert so die Übertebensfähigkeit der Zelle gegen oxidativen Stress wie beispielsweise UV-Strahlung. Aus den genannten Gründen ist G6PDH ein sehr wichtiges Enzym für die Regeneration der Haut, für die Synthese essentieller Substanzen und für den Schutz der Zellen vor oxidativen Stress.

Die Verwendung der erfindungsgemäßen Extrakte zur Herstellung von schützenden und aufbauenden Pflegemitteln ist prinzipiell für alle Zubereitungen möglich, die zur Prävention gegen Schädigungen oder bei Schädigungen der Haut und/oder Haare und damit in der Haut- und Haarpflege eingesetzt werden. Eine andere Verwendung auf diesem Gebiet ist die Applikation bei empfindlicher, durch Allergie oder anderen Ursachen geschädigter Haut. Die Schädigung der Haut kann dabei unterschiedlichste Ursachen haben.

Die erfindungsgemäßen Zubereitungen können zur Herstellung von kosmetischen und/oder dermopharmazeutischen Zubereitungen, wie beispielsweise Haarshampoos, Haarlotionen, Schaumbäder, Duschbäder, Cremes, Gele, Lotionen, alkoholische und wäßrig/alkoholische Lösungen, Emulsionen, Wachs/ Fett-Massen, Stiftpräparaten, Pudem oder Salben zum Einsatz kommen. Des weiteren können die erfindungsgemäßen Zubereitungen zur oralen Applikation auch in Tabletten, Dragees, Kapseln, Säfte, Lösungen und Granulate eingearbeitet sein.
Diese Zubereitungen können ferner als weitere Hilfs- und Zusatzstoffe milde Tenside, Ölkörper, Emulgatoren, Perlglanzwachse, Konsistenzgeber, Verdickungsmittel, Überfettungsmittel, Stabilisatoren, Polymere, Siliconverbindungen, Fette, Wachse, Lecithine, Phospholipide, biogene Wirkstoffe, UV-Uchtschutzfaktoren, Antioxidantien, Deodorantien, Antitranspirantien, Antischuppenmittel, Filmbildner, Quellmittel, Insektenrepellentien, Selbstbräuner, Tyrosininhibitoren (Depigmentierungsmittel), Hydrotrope, Solubilisatoren, Konservierungsmittel, Parfümöle, Farbstoffe und dergleichen enthalten.

### Tenside

Als oberflächenaktive Stoffe können anionische, nichtionische, kationische und/oder amphotere bzw. amphotere Tenside enthalten sein, deren Anteil an den Mitteln üblicherweise bei etwa 1 bis 70, vorzugsweise 5 bis 50 und insbesondere 10 bis 30 Gew.-% beträgt. Typische Beispiele für anionische Tenside sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Fettsäureethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für nichtionische Tenside sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofem die nichtionischen Tenside Polyglycoletherketten enthalten, können diese eine konventionelle, vorzugsweise jedoch eine eingeengte Homologenverteilung aufweisen. Typische Beispiele für kationische Tenside sind quartäre

Ammoniumverbindungen, wie beispielsweise das Dimethyldistearylammoniumchlorid, und Esterquats, insbesondere quatemierte Fettsäuretrialkanolaminestersalze. Typische Beispiele für amphotere bzw. zwitterionische Tenside sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten beispielsweise **J.Falbe (ed.), "Surfactants in Consumer Products", Springer Verlag, Berlin, 1987, S. 54-124 oder J.Falbe (ed.), "Katalysatoren, Tenside und Mineralöladditive", Thieme Verlag, Stuttgart, 1978, S. 123-217** verwiesen. Typische Beispiele für besonders geeignete milde, d.h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside, Fettsäureglucamide, Alkylamidobetaine, Amphoacetale und/oder Proteinfettsäurekondensate, letztere vorzugsweise auf Basis von Weizenproteinen.

### Ölkörper

Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen oder verzweigten C₆-C₂₂-Fettalkoholen bzw. Ester von verzweigten C₆-C₁₃-Carbonsäuren mit linearen oder verzweigten C₆-C₂₂-Fettalkoholen, wie z.B. Myristylmyristat, Myristylpalmitat, Myristylstearat, Myristylisostearat, Myristyrloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von C₁₈-C₃₈-Alkylhydroxycarbonsäuren mit linearen oder verzweigten C₆-C₂₂-Fettalkoholen (vgl. **DE 19756377 A1**), insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, wie z.B. Dicaprylyl Carbonate (Cetiol® CC), Guerbetcarbonate auf Basis von Fettalkoholen mit 6 bis 18, vorzugsweise 8 bis 10 C Atomen, Ester der Benzoesäure mit linearen und/oder verzweigten C₆-C₂₂-Alkoholen (z.B. Finsolv® TN). lineare oder verzweigte, symmetrische oder unsymmetrische Dialkylether mit 6 bis 22 Kohlenstoffatomen pro Alkylgruppe, wie z.B. Dicaprylyl Ether (Cetiol® OE), Ringöffnungsprodukte von epoxidierten Fettsäureestern mit Polyolen, Siliconöle (Cyclomethicone, Siliciummethicontypen u.a.) und/oder aliphatische bzw. naphthenische Kohlenwasserstoffe, wie z.B. wie Squalan, Squalen oder Dialkylcyclohexane in Betracht.

### Emulgatoren

Als Emulgatoren kommen beispielsweise nichtionogene Tenside aus mindestens einer der folgenden Gruppen in Frage:
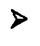 Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;
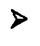 Alkyl- und/oder Alkenyloligoglykoside mit 8 bis 22 Kohlenstoffatomen im Alk(en)ylrest und deren ethoxylierte Analoga;
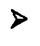 Anlagerungsprodukte von 1 bis 15 Mol Ethylenoxid an Ricinusöl und/oder gehärtetes Ricinusöl;
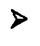 Anlagerungsprodukte von 15 bis 60 Mol Ethylenoxid an Ricinusöl und/oder gehärtetes Ricinusöl;
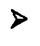 Partialester von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
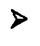 Partialester von Polyglycerin (durchschnittlicher Eigenkondensationsgrad 2 bis 8), Polyethylenglycol (Molekulargewicht 400 bis 5000), Trimethylolpropan, Pentaerythrit, Zuckeralkoholen (z.B. Sorbit), Alkylglucosiden (z.B. Methylglucosid, Butylglucosid, Laurylglucosid) sowie Polyglucosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
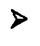 Mischester aus Pentaerythrit, Fettsäuren, Citronensäure und Fettalkohol gemäß **DE 1165574 PS** und/oder Mischester von Fettsäuren mit 6 bis 22 Kohlenstoffatomen, Methylglucose und Polyolen, vorzugsweise Glycerin oder Polyglycerin.
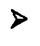 Mono-, Di- und Trialkylphosphate sowie Mono-, Di- und/oder Tri-PEG-alkylphosphate und deren Salze;
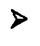 Wollwachsalkohole;
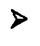 Polysiloxan-Polyalkyl-Polyether-Copolymere bzw. entsprechende Derivate;
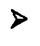 Block-Copolymere z.B. Polyethylenglycol-30 Dipolyhydroxystearate;
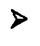 Polymeremulgatoren, z.B. Pemulen-Typen (TR-1,TR-2) von Goodrich;
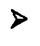 Polyalkylenglycole sowie
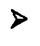 Glycerincarbonat.

Die Anlagerungsprodukte von Ethylenoxid und/oder von Propylenoxid an Fettalkohole, Fettsäuren, Alkylphenole oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus **DE 2024051 PS** als Rückfettungsmittel für kosmetische Zubereitungen bekannt.

Alkyl- und/oder Alkenyloligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 Kohlenstoffatomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Typische Beispiele für geeignete Partialglyceride sind Hydroxystearinsäuremonoglycerid, Hydroxystearinsäurediglycerid, Isostearinsäuremonoglycerid, Isostearinsäurediglycerid, Ölsäuremonoglycerid, Ölsäurediglycerid, Ricinolsäuremoglycerid, Ricinolsäurediglycerid, Linolsäuremonoglycerid, Linolsäurediglycerid, Linolensäuremonoglycerid, Linolensäurediglycerid, Enrcasäuremonoglycerid, Erucasäurediglycerid, Weinsäuremonoglycerid, Weinsäurediglycerid, Citronensäuremonoglycerid, Citronendiglycerid, Äpfelsäuremonoglycerid, Äpfelsäurediglycerid sowie deren technische Gemische, die untergeordnet aus dem Herstellungsprozeß noch geringe Mengen an Triglycerid enthalten können. Ebenfalls geeignet sind Anlagerungsprodukte von 1 bis 30, vorzugsweise 5 bis 10 Mol Ethylenoxid an die genannten Partialglyceride.

Als Sorbitanester kommen Sorbitanmonoisostearat, Sorbitansesquiisostearat, Sorbitandiisostearat, Sorbitantriisostearat, Sorbitanmonooleat, Sorbitansesquioleat, Sorbitandioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesquitartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitandimaleat, Sorbitantrimaleat sowie deren technische Gemische. Ebenfalls geeignet sind Anlagerungsprodukte von 1 bis 30, vorzugsweise 5 bis 10 Mol Ethylenoxid an die genannten Sorbitanester.

Typische Beispiele für geeignete Polyglycerinester sind Polyglyceryl-2 Dipolyhydroxystearate (Dehymuls® PGPH), Polyglycerin-3-Diisostearate (Lameform® TGI), Polyglyceryl-4 Isostearate (Isolan® GI 34), Polyglyceryl-3 Oleate, Diisostearoyl Polyglyceryl-3 Diisostearate (Isolan® PDI), Polyglyceryl-3 Methylglucose Distearate (Tego Care® 450), Polyglyceryl-3 Beeswax (Cera Bellina®), Polyglyceryl-4 Caprate (Polyglycerol Caprate T2010/90), Polyglyceryl-3 Cetyl Ether (Chimexane® NL), Polyglyceryl-3 Distearate (Cremophor® GS 32) und Polyglyceryl Polyricinoleate (Admul® WOL 1403) Polyglyceryl Dimerate Isostearate sowie deren Gemische. Beispiele für weitere geeignete Polyolester sind die gegebenenfalls mit 1 bis 30 Mol Ethylenoxid umgesetzten Mono-, Di- und Triester von Trimethylolpropan oder Pentaerythrit mit Laurinsäure, Kokosfettsäure, Talgfettsäure, Palmitinsäure, Stearinsäure, Ölsäure, Behensäure und dergleichen.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispiels-weise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin. Schließlich kommen auch Kationtenside als Emulgatoren in Betracht, wobei solche vom Typ der Esterquats, vorzugsweise methylquatemierte Difettsäuretriethanolaminester-Salze, besonders bevorzugt sind.

### Fette und Wachse

Typische Beispiele für Fette sind Glyceride, d.h. feste oder flüssige pflanzliche oder tierische Produkte, die im wesentlichen aus gemischten Glycerinestern höherer Fettsäuren bestehen, als Wachse kommen u.a. natürliche Wachse, wie z.B. Candelillawachs, Camaubawachs, Japanwachs, Espartograswachs, Korkwachs, Guarumawachs, -Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse in Frage. Neben den Fetten kommen als Zusatzstoffe auch fettähnliche Substanzen, wie Lecithine und Phospholipide in Frage. Unter der Bezeichnung Lecithine versteht der Fachmann diejenigen Glycero-Phospholipide, die sich aus Fettsäuren, Glycerin, Phosphorsäure und Cholin durch Veresterung bilden. Lecithine werden in der Fachwelt daher auch häufig als Phosphatidylcholine (PC). Als Beispiele für natürliche Lecithine seien die Kephaline genannt, die auch als Phosphatidsäuren bezeichnet werden und Derivate der 1,2-Diacyl-sn-glycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage.

### Periglanzwachse

Als Periglanzwachse kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxysubstituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether, Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

### Konsistenzgener und Verdickungsmittel

Als Konsistenzgeber kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete Verdickungsmittel sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, (z.B. Carbopole® und Pemulen-Typen von Goodrich; Synthalene® von Sigma; Keltroi-Typen von Kelco; Sepigel-Typen von Seppic; Safcare-Typen von Allied Colloids), Polyacrylamide, Polymere, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

### Überfettungsmittel

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

### Stabilisatoren

Als Stabilisatoren können Metallsalze von Fettsäuren, wie z.B. Magnesium-, Aluminium- und/oder Zinkstearat bzw. -ricinoleat eingesetzt werden.

### Polymere

Geeignete katronische Polymere sind beispielsweise kationische Cellulosederivate, wie z.B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinylimidazol-Polymere, wie z.B. Luviquat® (BASF), Kondensationsprodukte von Polyglycolen und Aminen, quatemierte Kollagenpolypeptide, wie beispielsweise Lauryldimonium Hydroxypropyl Hydrolyzed Collagen (Lamequat®L/Grünau), quatemierte Weizenpolypeptide, Polyethylenimin, kationische Siliconpolymere, wie z.B. Amodimethicone, Copolymere der Adipinsäure und Dimethylaminohydroxypropyldiethylentriamin (Cartaretine®/Sandoz), Copolymere der Acrylsäure mit Dimethyl-diallylammoniumchlorid (Merquat® 550/Chemviron), Polyaminopolyamide, wie z.B. beschrieben in der **FR 2252840 A** sowie deren vemetzte wasserlöslichen Polymere, kationische Chitinderivate wie beispielsweise quatemiertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quatemierte Ammoniumsalz-Polymere, wie z.B. Mirapol® A-15, Mirapol® AD-1, Mirapol® AZ-1 der Firma Miranol.

Als anionische, zwitterionische, amphotere und nichtionische Polymere kommen beispielsweise Vinylacetat/Crotonsäure-Copolymere, Vinylpyrrolidon/Vinylacrylat-Copolymere, Vinylacetat/Butylmaleat/Isobomylacrylat-Copolymere, Methylvinylether/Maleinsäureanhydrid-Copolymere und deren Ester, unvemetzte und mit Polyolen vemetzte Polyacrylsäuren, Acrylamidopropyltrimethylammoniumchlorid/Acrylat Copolymere, Octylacrylamid/Methylmeth-acrylat/tertButylaminoethylmethacrytat/2-Hydroxypropylmethacrylat-Copolymere, Polyvinylpyrrolidon, Vinylpyrrolidon/Vinylacetat-Copolymere, Vinylpyrrolidon/ Dimethylaminoethylmethacrylat/Vinylcaprolactam-Terpolymere sowie gegebenenfalls derivatisierte Celluloseether und Silicone in Frage. Weitere geeignete Polymere und Verdickungsmittel sind in **Cosm.Toll. 108, 95 (1993)** aufgeführt.

### Siliconverbindungen

Geeignete Siliconverbindungen sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt. Eine detaillierte Übersicht über geeignete flüchtige Silicone findet sich zudem von Todd et al. in **Cosm.Toil. 91, 27 (1976).**

### Antioxidantien

Neben den genannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-Apalmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Hamsäure und deren Derivate, Mannose und deren Derivate, Superoxid-Dismutase, Zink und dessen Derivate (z.B. ZnO, ZnSO₄) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

### Biogene Wirkstoffe

Unter biogenen Wirkstoffen sind im Rahmen der Erfindung zusätzlich solche zu verstehen, die nicht aus der Pflanze Argania spinosa stammen, wie beispielsweise Tocopherolacetat, Tocopherolpalmitat, Ascorbinsäure, (Desoxy)Ribonucleinsäure und deren Fragmentierungsprodukte, Retinol, Bisabolol, Allantoin, Phytantriol, Panthenol, AHA-Säuren, Aminosäuren, Ceramide, Pseudoceramide, essentielle Öle, weitere Pflanzenextrakte und zusätzliche Vitaminkomplexe.

### Deodorantien und keimhemmende Mittel

Kosmetische Deodorantien (Desodorantien) wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend enthalten Deodorantien Wirkstoffe, die als keimhemmende Mittel, Enzyminhibitoren, Geruchsabsorber oder Geruchsüberdecker fungieren. Als keimhemmende Mittel sind grundsätzlich alle gegen grampositive Bakterien wirksamen Stoffe geeignet, wie z. B. 4-Hydroxybenzoesäure und ihre Salze und Ester, N-(4-Chlorphenyl)-N'-(3,4 dichlorphenyl)hamstoff, 2,4,4'-Trichlor-2'-hydroxydiphenylether (Triclosan), 4-Chlor-3,5-dimethyl-phenol, 2,2-Methylen-bis(6-brom-4-chlorphenol), 3-Methyl-4-(1-methylethyl)-phenol, 2-Benzyl-4-chlorphenol, 3-(4-Chlorphenoxy)-1,2-propandiol, 3-lod-2-propinylbutylcarbamat, Chlorhexidin, 3,4,4'-Trichlorcarbanilid (TTC), antibakterielle Riechstoffe, Thymol, Thymianöl, Eugenol, Nelkenöl, Menthol, Minzöl, Famesol, Phenoxyethanol, Glycerinmonocaprinat, Glycerinmonocaprylat, Glycerinmonolaurat (GML), Diglycerinmonocaprinat (DMC), Salicylsäure-N-alkylamide wie z. B. Salicylsäure-n-octylamid oder Salicylsäure-n-decylamid.

Als Enzyminhibitoren sind beispielsweise Esteraseinhibitoren geeignet Hierbei handelt es sich vorzugsweise um Trialkylcitrate wie Trimethylcitrat, Tripropylcitrat, Triisopropylcitrat, Tributylcitrat und insbesondere Triethylcitrat (Hydagen® CAT). Die Stoffe inhibieren die Enzymaktivität und reduzieren dadurch die Geruchsbildung. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbnonsäuren und deren Ester wie beispielsweise Citronensäure, Äpfelsäure, Weinsäure oder Weinsäurediethylester, sowie Zinkglycinat.

Als Geruchsabsorber eignen sich Stoffe, die geruchsbildende Verbindungen aufnehmen und weitgehend festhalten können. Sie senken den Partialdruck der einzelnen Komponenten und verringern so auch ihre Ausbreitungsgeschwindigkeit. Wichtig ist, daß dabei Parfüms unbeeinträchtigt bleiben müssen. Geruchsabsorber haben keine Wirksamkeit gegen Bakterien. Sie enthalten beispielsweise als Hauptbestandteil ein komplexes Zinksalz der Ricinolsäure oder spezielle, weitgehend geruchsneutrale Duftstoffe, die dem Fachmann als "Fixateure" bekannt sind, wie z. B. Extrakte von Labdanum bzw. Styrax oder bestimmte Abietinsäurederivate. Als Geruchsüberdecker fungieren Riechstoffe oder Parfümöle, die zusätzlich zu ihrer Funktion als Geruchsüberdecker den Deodorantien ihre jeweilige Duftnote verleihen. Als Parfümöle seien beispielsweise genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen sowie Harzen und Balsamen. Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, p-tert.-Butylcyclohexylacetat, Linalylacetat, Phenylethylacetat, Linalylbenzoat, Benzylformiat, Allylcyclohexylpropionat, Styrallylpropionat und Benzylsalicylat. Zu den Ethem zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätterol, Lindenblütenöl, Wacholderbeerenöl, Vetiveröl, Olibanöl, Galbanumöl, Labdanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilat, Irotyl und Floramat allein oder in Mischungen, eingesetzt.

Antitranspirantien (Antiperspirantien) reduzieren durch Beeinflussung der Aktivität der ekkrinen Schweißdrüsen die Schweißbildung, und wirken somit Achselnässe und Körpergeruch entgegen. Wässrige oder wasserfreie Formulierungen von Antitranspirantien enthalten typischerweise folgende Inhaltsstoffe:
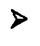 adstringierende Wirkstoffe,
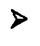 Ölkomponenten,
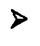 nichtionische Emulgatoren,
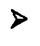 Coemulgatoren,
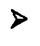 Konsistenzgeber,
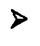 Hilfsstoffe wie z. B. Verdicker oder Komplexierungsmittel und/oder
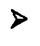 nichtwässrige Lösungsmittel wie z. B. Ethanol, Propylenglykol und/oder Glycerin.

Als adstringierende Antitranspirant-Wirkstoffe eignen sich vor allem Salze des Aluminiums, Zirkoniums oder des Zinks. Solche geeigneten antihydrotisch wirksamen Wirkstoffe sind z.B. Aluminiumchlorid, Aluminiumchlorhydrat, Aluminiumdichlorhydrat, Aluminiumsesquichlorhydrat und deren Komplexverbindungen z. B. mit Propylenglycol-1,2. Aluminiumhydroxyallantoinat, Aluminiumchloridtartrat, Aluminium-Zirkonium-Trichlorohydrat, Aluminium-Zirko-nium-tetrachlorohydrat Aluminium-Zirkonium-pentachlorohydrat und deren Komplexverbindungen z. B. mit Aminosäuren wie Glycin. Daneben können in Antitranspirantien übliche öllösliche und wasserlösliche Hilfsmittel in geringeren Mengen enthalten sein. Solche öllöslichen Hilfsmittel können z.B. sein:
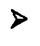 entzündungshemmende, hautschützende oder wohlriechende ätherische Öle,
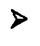 synthetische hautschützende Wirkstoffe und/oder
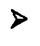 öllösliche Parfümöle.

Übliche wasserlösliche Zusätze sind z.B. Konservierungsmittel, wasserlösliche Duftstoffe, pH-Wert-Stellmittel, z.B. Puffergemische, wasserlösliche Verdickungsmittel, z.B. wasserlösliche natürliche oder synthetische Polymere wie z.B. Xanthan-Gum, Hydroxyethylcellulose, Polyvinylpyrrolidon oder hochmolekulare Polyethylenoxide.

### Filmbildner

Gebräuchliche Filmbildner sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen.

### Antischuppenwirkstoffe

Als Antischuppenwirkstoffe kommen Pirocton Olamin (1-Hydroxy-4-methyl-6-(2,4,4-trimythylpentyl)-2-(1H)-pyridinonmonoethanolaminsalz), Baypival® (Climbazole), Ketoconazol®, (4-Acetyl-1-{-4-[2-(2,4-dichlorphenyl) r-2-(1H-imidazol-1-ylmethyl)-1,3-dioxylan-c-4-ylmethoxyphenyl}piperazin, Ketoconazol, Elubiol, Selendisulfid, Schwefel kolloidal, Schwefelpolyehtylenglykolsorbitanmonooleat, Schwefelrizinolpolyehtoxylat, Schwfel-teer Destillate, Salicylsäure (bzw. in Kombination mit Hexachlorophen), Undexylensäure Monoethanolamid Sulfosuccinat Na-Salz, Lamepon® UD (Protein-Undecylensäurekondensat), Zinkpyrithion, Aluminiumpyrithion und Magnesiumpyrithion / Dipyrithion-Magnesiumsulfat in Frage.

### Quellmittel

Als Quellmittel für wäßrige Phasen können Montmorillonite, Clay Mineralstoffe, Pemulen sowie alkylmodifizierte Carbopoltypen (Goodrich) dienen. Weitere geeignete Polymere bzw. Quellmittel können der Übersicht von R.Lochhead in **Cosm.Toil. 108,95** (**1993**) entnommen werden.

### Insekten-Repellentien

Als Insekten-Repellentien kommen N,N-Diethyl-m-toluamid, 1,2-Pentandiol oder Ethyl Butylacetylaminopropionate in Frage

### Selbstbräuner und Depigmentierungsmittel

Als Selbstbräuner eignet sich Dihydroxyaceton. Als Tyrosinhinbitoren, die die Bildung von Melanin verhindern und Anwendung in Depigmentierungsmitteln finden, kommen beispielsweise Arbutin, Ferulasäure, Kojisäure, Cumarinsäure und Ascorbinsäure (Vitamin C) in Frage.

### Hydrotrope

Zur Verbesserung des Fließverhaltens können ferner Hydrotrope, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind
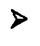 Glycerin;
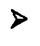 Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
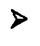 technische Oligoglyceringemische mit einem Eigenkondensationsgrad von 1,5 bis 10 wie etwa technische Diglyceringemische mit einem Diglyceringehalt von 40 bis 50 Gew.-%;
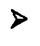 Methyolverbindungen, wie insbesondere Trimethylolethan, Trimethylolpropan, Trimethylolbutan, Pentaerythrit und Dipentaerythrit;
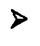 Niedrigalkylglucoside, insbesondere solche mit 1 bis 8 Kohlenstoffen im Alkylrest, wie beispielsweise Methyl- und Butylglucosid;
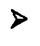 Zuckeralkohole mit 5 bis 12 Kohlenstoffatomen, wie beispielsweise Sorbit oder Mannit,
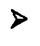 Zucker mit 5 bis 12 Kohlenstoffatomen, wie beispielsweise Glucose oder Saccharose;
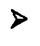 Aminozucker, wie beispielsweise Glucamin;
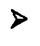 Dialkoholamine, wie Diethanolamin oder 2-Amino-1,3-propandiol.

### Konservierungsmittel

Als Konservierungsmittel eignen sich beispielsweise Phenoxyethanol, Formaldehydlösung, Parabene, Pentandiol oder Sorbinsäure sowie die in Anlage 6, Teil A und B der Kosmetikverordnung aufgeführten weiteren Stoffklassen.

### Parfümöle

Als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Meter, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benrylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benrylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyrlamenaldehyd, Hydroxycitronellal, Lilial und Bourgeonal, zu den Ketonen z.B. die Jonone, α-Isomethylionon und Methylcedrylketon, zu den Alkoholen Anethol, Citronellol, Eugenol, Isoeugenol, Geraniol, Unalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Unalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evemyl, Iraldein gamma, Phenylessigsäure, Geranylacetat, Benzylacetat, Rosenoxid, Romilllat, Irotyl und Floramat allein oder in Mischungen, eingesetzt.

### Farbstoffe

Als Farbstoffe können die für kosmetische Zwecke geeigneten und zugelassenen Substanzen verwendet werden, wie sie beispielsweise in der Publikation "**Kosmetische Färbemittel" der Farbstoff kommission der Deutschen Forschungsgemeinschaft, Verlag Chemie, Weinheim, 1984, S.81-106** zusammengestellt sind, Diese Farbstoffe werden üblicherweise in Konzentrationen von 0,001 bis 0,1 Gew.-%, bezogen auf die gesamte Mischung, eingesetzt.

### Beispiele

### 1. Beispiel: Extraktion der Pflanzen mit destilliertem Wasser

0,2 kg entfettete Argania spinosa Samenkeme erhalten aus dem Rückstand der Extraktion zur Ölgewinnung wurden in ein Glasgefäß überführt und mit 21 destilliertem Wasser aufgegossen. Die Mischung wurde bei Raumtemperatur für zwei Stunden gerührt. Der pH-Wert der Lösung lag zwischen 6,2 und 6,0. Anschließend wurde die Mischung 15 min bei einer Geschwindigkeit von 5000 g zentrifugiert Die überstehende Flüssigkeit wurde durch Filtration an Tiefenfilter mit einer mittleren Porosität von 450 nm (von der Firma Seitz, Bordeaux Frankreich) vom Rückstand getrennt. Die Ausbeute an nativen Proteinen berechnet auf Trockengewicht (nach N x 6,25) betrug 35 bis 55 Gew.-%.

### 2. Beispiel: Aufarbeitung des Extraktes durch Thermo-Reinigung

Beispiel 1 wurde wiederholt, die Aufreinigung jedoch durch das Thermo-Reinigungsverfahren durchgeführt Hierzu wurde die überstehende Flüssigkeit nach dem wie unter Beispiel 1 beschriebenen Zentrifugieren für 30 min auf 80-100 °C erhitzt, was zur Ausfällung der hitzeinstabilen nativen Proteine führte und anschließend auf Raumtemperatur abgekühlt. Anschließend wurde die Mischung 15 min bei einer Geschwindigkeit von 5000 g zentrifugiert und durch Filtration an Tiefenfilter mit einer mittleren Porosität von 220 nm (von der Firma Seitz, Bordeaux Frankreich) vom Rückstand getrennt.

Durch Chromatographie an Superose 12HR konnten drei Hauptfraktionen an native Proteinen isoliert werden. Diese hatten Molekulargewichte in folgenden Bereichen:

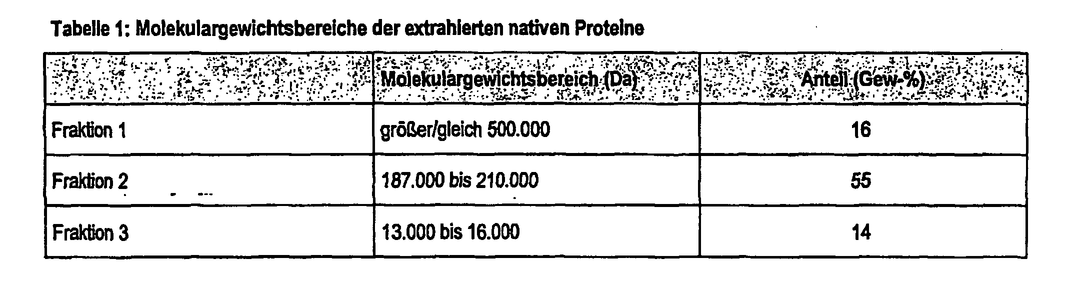

### 3. Beispiel: Extraktion mit Anreicherung der Fraktion 2

Beispiel 1 wurde bis zur Zentrifugation wiederholt. 1,6 Liter dieses Proteinextraktes wurden in einen Reaktor gegeben und unter Rühren durch Zugabe von 4N Schwefelsäure auf einen pH-Wert von 4,5 eingestellt Die Mischung wurde 15 bis 30 min. gerührt. Anschließend wurde die Mischung 15 min bei einer Geschwindigkeit von 5000 g zentrifugiert. Der erhaltene Rückstand war angereichert mit der Fraktion im Molekulargewichtsbereich zwischen 187.000 und 210.000 Da insbesondere mit Proteinen eines Molekulargewichtes von 200.000 Da und der Überstand enthielt die Proteinfraktion mit dem niedrigsten Molekulargewicht. Der Rückstand wurde mit 160 ml Wasser aufgenommen und der pH-Wert unter Rühren durch Zugabe von 4N NaOH auf pH 6,1 eingestellt. Die so erhaltene Lösung wurde erneut unter den beschriebenen Bedingungen zentrifugiert und durch Gefriertrocknung getrocknet. Durch diese Anreicherung konnte ein Extrakt erhalten werden, der 70-85 Gew.-% der nativen Proteine nach Fraktion 2 aus Beispiel 2 enthielt. Der gesamte Proteinanteil im erhaltenen getrockneten Extrakt betrug nach dieser Extraktionsmethode 60-85 Gew.-%.

### 4. Beispiel: Extraktion mit Anreicherung der Fraktion 3

1,48 Liter des Rückstandes aus Beispiel 3 wurde durch Filtration an Tiefenfilter mit einer mittleren Porosität von 220 nm (von der Firma Seitz, Bordeaux Frankreich) vom Rückstand getrennt und anschließend gefriergetrocknet Durch diese Anreicherung konnte ein Extrakt erhalten werden, der 21-40 Gew.-% der nativen Proteine nach Fraktion 3 aus Beispiel 2 enthielt. Der gesamte Proteinanteil im erhaltenen getrockneten Extrakt betrug nach dieser Extraktionsmethode 40-50 Gew.-%, nachgewiesen durch ein UV-Chromatogramm.

### 5. Beispiel: Test zur Feuchtigkeitsregulierung der Haut

Hintergrund: In der Epidermis menschlicher Haut findet sich die Hornschicht (das Stratum corneum), sein Wassergehalt sichert ihm einerseits seine Elastizität und bestimmt andererseits Menge sowie vielleicht auch Größe der an sich mikroskopisch kleinen abgeschilferten Hornschuppen.

Methode: Proben aus der plastischen Chirurgie wurden für diese feuchtigkeitsregulierenden Test verwendet. Zwei unterschiedliche Bedingungen wurden getestet. Zum einen wurde normale Haut als Kontrolle untersucht und zum anderen wurde eine Hautprobe, deren Oberfläche geschädigt wurde behandelt und untersucht. Das Stratum comeum aus diesen Hautproben wurde für eine Stunde in einer 5%igen Lösung von Natrium-Laurylsulfat getränkt, anschließend bei Raumtemperatur getrocknet und auf Gittern aufgezogen in hermetisch abgeriegelten Kammern mit definierter relativer Feuchtigkeit (44 %, gesättigte Lösung von Kaliumcarbonat) gelagert und standardisiert. Jede Probe des Stratum corneums wurde unter drei Bedingungen vergleichend getestet.
1) ohne Behandlung;
2) Behandlung mit Placebo;
3) Behandlung mit einer Zubereitung die aus einem Bindemittel besteht (Hydrogel LS von der Firma Laboratoire Sérobiologique LS), enthaltend 5 Gew.-% Extrakt nach Beispiel 4.

Es wurde jeweils 2 mg/cm² Placebo bzw. Zubereitung nach 3) auf die externe Oberfläche aufgetragen. Als Placebo diente das Bindemittel (Hydrogel LS der Firma Laboratoire Sérobiologique LS) ohne die beschriebene Zubereitung, also ohne Pflanzenextrakt.

Die feuchtigkeitsregulierende Aktivität der erfindungsgemäßen nativen Proteine in oben beschriebener Zubereitung wurden bestimmt durch den Verlust an Feuchtigkeit im Stratum corneum während einer Zeitspanne von 24 Stunden, angegeben in mg/h/cm² im Vergleich zur Placebo Behandlung.

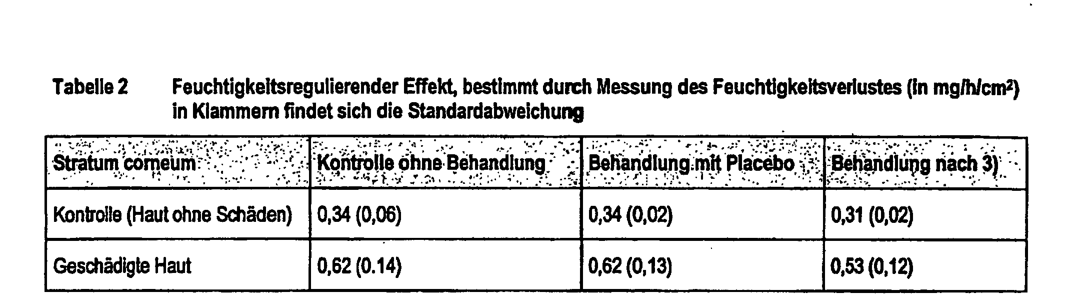

Die Ergebnisse der Untersuchungen zeigen eine feuchtigkeitsregulierende Aktivität der nativen Proteine aus Argania spinosa. Die Hautproben, weiche mit Zubereitungen nach 3) behandelt wurden zeigten einen deutlich geringeren Verlust an Feuchtigkeit im Verlauf von 24 Stunden als unbehandelte Hautproben. Der Unterschied war bei bereits geschädigter Haut noch deutlicher zu erkennen als bei Haut ohne Schäden.

### 6. Hautstraffende Effekte (Dynamic spring rate)

Hintergrund: Das Prinzip dieser Methode besteht darin, eine Verschiebung oder Verlagerung der Haut als Antwort auf eine geringe sinusoidale Kraft, welche parallel zur Hautoberfläche aufgewandt wird, zu bestimmen. Diese Kraft wird mit Hilfe eines gastragenden Elektrodynamometers erzeugt Der untersuchte Parameter ist der sogenannte "dynamic spring rate" (DSR) der das Verhältnis von aufgewendeter Kraft zur Verschiebung der Haut ausdrückt. Je größer die Verschiebung der Haut im Verhältnis zur aufgewendeten Kraft, desto geschmeidiger ist die Haut und umgekehrt.

Eine hohe Verschiebbarkeit der Haut nach der Behandlung mit den zu untersuchenden Proben wird durch eine Verringerung des DSR ausgedrückt und eine Erhöhung des dynamic spring rates zeigt eine straffende, stabilisierende Wirkung der zu untersuchenden Probe auf die Haut.

Methode: Die Hautprobe wurde auf einen Objektträger montiert und befestigt. Die befestigte Haut wurde über zwei Stunden in einer Athmosphäre mit kontrollierter Luftfeuchtigkeit (33 %) und konstanter Temperatur (T = 20°C) äquilibriert. Anschliessend wurden die mechanischen Eigenschaften unter drei Bedingungen vergleichend bestimmt. Die Ergebnisse sind dargestellt als die Entwicklung des DSR nach 90 Minuten.

**Tabelle 3**

| **Hautstraffender und stabilisierender Effekt, bestimmt durch Messung des dynamic spring rates (DSR) der das Verhältnis von aufzuwendender Kraft zu Verschiebung der Haut ausdrückt.** | |
|---|---|
| | **DSR nach 90 min.** |
| Kontrolle ohne Behandlung | 98 % |
| Placebo Hydrogel ohne Argania spinosa Extrakt | 116 % |
| Hydrogel enthaltend 2 Gew.-% Extrakt nach Beispiel 3 | 135% |
| Hydrogel enthaltend 6 Gew.-% Extrakt nach Beispiel 4 | 142 % |

Es wurden jeweils 4 mg/cm² Hydrogel aufgetragen.

Im Vergleich zu den Placebo und Kontrollversuchen war die Erhöhung des DSR-Wertes für Hydrogel enthaltend Extrakte aus Argania spinosa signifikant Diese erhöhten Werte verdeutlichen, dass für ein Verschiebung der befestigten Haut nach der Behandlung mit den zu untersuchenden Proben eine größere Kraft aufgewendet werden muss als ohne Extrakt aus Argania spinosa und diese Werte verdeutlichen damit eine straffende und stabilisierende Wirkung der erfindungsgemäßen Extrakte auf die Haut.

### 7. Wirkung auf die Überlebensaktivität humaner Fibroblasten

Für die Beurteilung der Zellaktivität gibt es wesentliche Marker, zu denen MIT, Proteine und Glutathion zählen.

Das Überleben wurde durch die folgenden Gehalte ausgewertet:
- Rate des metabotisierten MTT (Methyl Thiazolyl Tetrazolium); Die Aktivität der Mitochondrien wird über den MTT-Test bestimmt. MTT wird durch ein Enzym der Respirationskette, Succinatdehydrogenase, in Formazan reduziert (Denizot F, Lang R, Rapid colorimetric assay for cell growth and survival. J. Immunol. Methods, 89, 271-277,1986).
- von Proteinen; Die Proteinkonzentration der Zellen wurde bestimmt nach Bradford (Bradford M.M. A rapid and sensitive method for the quantitation of microgram quantities of protein utilizing the principle of protein-dye binding. Anal. Biochem. (1977) vol 72, pp 248-254)
- von Glutathion (GSH), ein direkt von der Zelle erzeugtes Peptid, zur Bekämpfung von oxydativem Stress oder von verschiedenen Schmutzstoffen, wie zum Beispiel Schwermetalle. Seine Synthese erfordert ATP als Energiequelle. GSH wurde bestimmt nach Hissin (Hissin P.J., Hilf R. A fluorometric method for determination of oxidised and reduced Glutathione in tissues. Analytical Biochemistry (1977) vol 74, pp 214-226).

Glutathion (GSH) ist ein Peptid, das von Zellen produziert wird, um die Zelle vor oxidativem Stress oder Schwermetallen wie beispielsweise Blei oder Quecksilber zu schützen. Die drei Aminosäuren die bei der reduzierten Form von GSH involviert sind, sind wiederum verbunden mit spezifischen cytoplasmatischen Enzymen, die ATP benötigen.

Die Steigerung des GSH-Levels hat einen positiven Einfluss auf die Aktivität der Glutathion-S-transferase, die ein entgiftendes Enzym darstellt.

Methode: Menschliche Fibroblaste wurden in ein Nährmedium (DMEM = Dulbecco Minimum Essential Medium von der Firma Life Technologie Sarl) mit 10 % fötalem Kälberserum (von der Firma Dutcher) geimpft und 24 Stunden lang bei 37° C in einer 5 %igen CO₂-Athmosphäre inkubiert.

Das Medium wurde danach durch ein Sub-Optimum-Medium (ohne SVF) ersetzt, das verschiedene Extrakte in verschiedenen Konzentrationen (0,01; 0,03 und 0,1 Gew.-%) nach der Beschreibung der Erfindung enthielt..

Die Ergebnisse werden im Verhältnis zu einer extraktfreien Formulierung für Protein, MTT und GSH in das Verhältnis gestellt und in einem Prozentsatz im Verhältnis zum unbehandelten Kontrollmittel angegeben als Durchschnittswert +/- SEM (Fehlertyp des Durchschnitts) ausgedrückt.

**Tabelle 4:**

| **Zellüberlebens - Test - (Ergebnisse in % basierend auf der Kontrolle ohne Extrakt (Mittelwert von 2 Assays in dreifacher Ausführung)** | | | | |
|---|---|---|---|---|
| Konzentration in Gew.-% | | MIT | Proteine | GSH / Proteine |
| Kontrolle | 0 | 100 | 100 | 100 |
| Extrakt nach Beispiel 3 | 0,03 | 108 | 104 | 107 |
| | 0,1 | 100 | 102 | 152 |
| Extrakt nach Beispeil 4 | 0,01 | 99 | 90 | 128 |
| | 0,03 | 118 | 91 | 263 |

Die Tabelle gibt jeweils die Mitochondrienaktivität über die MTT, Proteingehalte und die GSH-Gehalte an, die nach drei Tagen für verschiedene Konzentrationen an Extrakten gemessen wurden. Eine Fraktion nativer Proteine der Argania spinosa nach Beispiel 4 mit einer Konzentration von 0,03 Gew.% hat die Mitochondrienaktivität danach erheblich erhöht (+ 18 %). Eine Fraktion nativer Proteine aus Argania spinosa nach Beispiel 4 mit einer Konzentration von nur 0,03 % ist in der Lage den GSH-Gehalt in menschlichen Fibroblasten um 163 % zu erhöhen.

Diese Ergebnisse zeigen, dass die ursprünglichen oder hydrolysierten Proteinextrakte von Argania spinosa hohe Fähigkeiten zur Verbesserung des Metabolismus (Synthese von Proteinen und des Glutathion) durch die menschlichen Fibroblaste aufweisen, was deutlich eine energiespendende, summierende und ''Anti-Älterungs"- Aktivität dieser Extrakte angibt.

### 8. Beispiel: Zellschutzwirkung gegen UVA an in vitro gezüchteten menschlichen Fibroblasten

Hintergrund: UVA-Strahlen dringen bis in die Dermis ein, wo sie zu Oxidationsstress führen, was durch eine Lipoperoxidation der Zytoplasmamembranen nachgewiesen wird.

Die Lipoperoxide werden zu Malonaldialdehyd abgebaut, der viele biologische Moleküle wie Proteine und Nukleinbasen vernetzen wird (Enzymhemmung bzw. Mutagenese).

Glutathione (GSH) ist ein Peptid, welches direkt von den Zellen produziert wird um oxidativem Stress oder schädigenden Umwelteinflüssen wie zum Beispiel einer erhöhten Quecksilber- oder Bleibelastung entgegen zu wirken. Der Gehalt an GSH wurde bestimmt nach der Methode von Hissin, beschrieben in Anal. Biochem., 74, 214-226, 1976.

Methode: Zur Durchführung dieser Tests wurde ein definiertes Kulturmedium (DMEM) mit 10 % fötalem Kälberserum mit den Fibroblasten beimpft und der Pflanzenextrakt (in dem definierten Medium mit 2% Serum) 72 Stunden nach dem Beimpfen zugegeben.

Nach 48 stündiger Inkubation bei 37°C und einem CO₂-Gehalt von 5 % wurde das Kulturmedium durch eine Kochsalzlösung ersetzt und die Fbroblasten wurden mit einer UVA-Dosis bestrahlt (20 J/cm²; Röhren: MAZDA FLUOR TFWN40).

Nach der Beendigung der Bestrahlung wurde der Gehalt an Zellproteinen und der Anteil an GSH bestimmt sowie der MDA-Spiegel (Malonaldialdehyd-Spiegel) in der überstehenden Saline-Lösung quantitativ durch Reaktion mit Thiobarbitursäure bestimmt. Die Ergebnisse sind angegeben in Prozent im Vergleich zur Kontrolle ohne Bestrahlung.

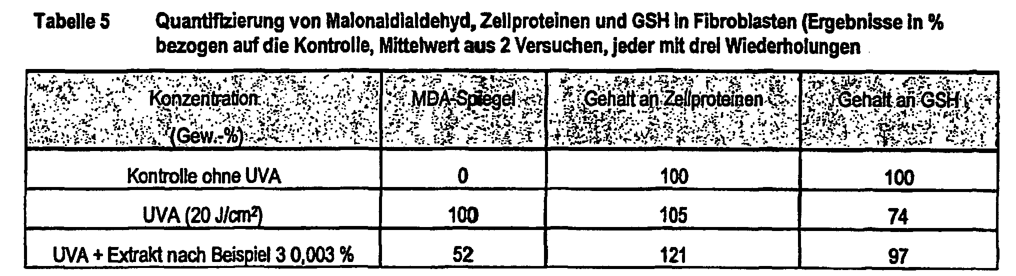

Die Ergebnisse aus der Tabelle 5 zeigen, dass die erfindungsgemäßen Extrakte aus der Pflanze Argania spinosa signifikant den Grad an MDA in humanen Fibroblasten, welcher durch UVA-Strahlen induziert wird, reduzieren. Des weiteren ergibt sich eine hohe Aktivität, den Anteil an GSH in humanen Fibroblasten nach einer Bestrahlung mit UVA-Strahlung relativ konstant zu halten. Diese Ergebnisse zeigen eine hohe Kapazität von Proteinfraktionen aus Argania spinosa schädliche Effekte eines oxidativen Stresses auf der Haut zu reduzieren.

### 9. Beispiel: Entzündungshemmende Eigenschaften in vitro- UVB Lichtschutz

### Zellschutzwirkung gegen UVB an in vitro gezüchteten menschlichen Keratinozyten

Hintergrund: UVB-Strahlen (von 280 bis 320 nm) lösen durch Aktivierung eines Enzyms, nämlich Phospholipase A2 oder PLA2, die Arachidonsäure aus den Phospholipiden der Plasmamembran entfernt, eine Entzündung (Erythem, Ödem) aus. Arachidonsäure ist die Vorstufe der Prostaglandine, die eine Entzündung und eine Zellmembranschädigung verursachen; die Prostaglandine E2 (= PGE2) werden durch die Cyclooxygenase gebildet. Dieser Membranstress wird durch die Freisetzung des Cytoplasmaenzyms Lactat Dehydrogenase (LDH) angezeigt. Der Effekt von UVB-Strahlung wurde an Keratinocyten in vitro untersucht indem die Freisetzung des Cytoplasmaenzyms LDH (Lactat Dehydrogenase) bestimmt wurde. Dieses Enzym dient als Marker für eine Zellschädigung.

Methode:. Zur Durchführung der Tests wurde ein definiertes Medium (DMEM), das 10 % fötales Kälberserum enthält, mit den Keratinozyten beimpft und der Pflanzenextrakt (mit Saline-Lösung verdünnt) 72 Stunden nach dem Beimpfen zugegeben.

Die Keratinozyten wurden sodann mit einer UVB-Dosis bestrahlt (50 mJ/cm² - Röhren: DUKE GL40E).

Nach weiterer 1 tägiger inkubation bei 37 °C und bei 5 % CO₂ wurde der LDH- und der PGE2-Gehalt im Überstand bestimmt. Der Gehalt von LDH- (Lactatdehydrogenase) wurde mittels einer Enzymreaktion bestimmt (verwendetes kit zur Untersuchung des LDH Gehaltes von der Firma Roche) Der Gehalt an PGE2 wurde mit einem ELISA-Test (ELISA Kit der Firma Roche) bestimmt Nach der Trypsin-Behandlung wurden die Zellen zentrifugiert und ausgezählt.

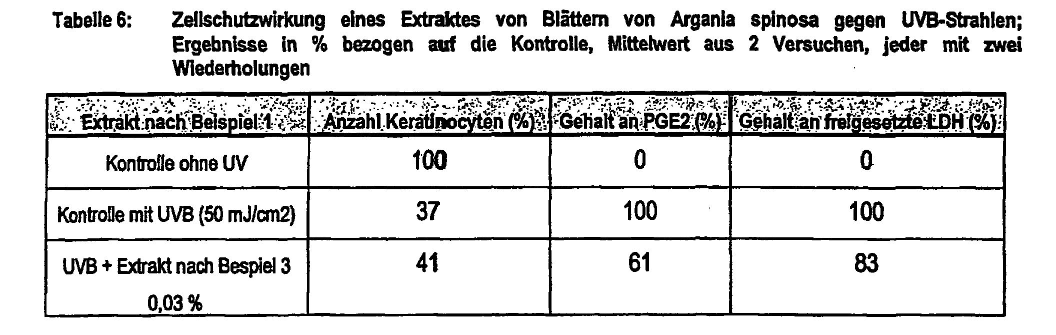

Die Ergebnisse dieser Tests belegen, dass eine erfindungsgemäße Proteinfraktion der Pflanze Argania spinosa den Effekt von UVB-Strahlung auf die Anzahl an Keratinocyten reduziert. Es zeigt sich eine Verringerung des Gehalts an freigesetzte LDH im Cytoplasma und ein Verringerung des PGE2-Gehaltes. Die beschriebenen Proteinextrakte zeigen demnach die Fähigkeit, die durch UVB-Strahlung hervorgerufene Schädigung an Zellmembranen zu reduzieren und zeigen eine hemmende Wirkung gegen Entzündungen, die durch UVB Strahlung induziert werden.

### 10. Beispiel: Bestimmung der enzymatischen Aktivität der Glucose-6-phosphate Dehydrogenase

Hintergrund: Das Ziel dieses Tests ist es, die stimulierenden Eigenschaften auf die enzymatische Aktivität der Glucose-6-phosphate Dehydrogenase (G6PDH) zu untersuchen, welches die Hautalterung beschleunigen kann. Glucose-6-phosphat Dehydrogenase katalysiert den ersten Schritt des oxidativen Zweiges des Pentosephosphat-Weges. In diesem ersten Schritt wird Glucose-6-phosphat zu 6-Phosphono-δ-lacton unter Einwirkung von NADP. Dieses Coenzym wird bei dieser Oxidation zu NADPH2 reduziert. Die reduzierte Form dieses Coenzyms kann viele enzymatische Reaktionen wie beispielsweise Recycling von Glutthion oder Upidsynthese katalysieren. Des weiteren produziert der Pentosephosphat-Weg eine essentielle Komponenten für den Aufbau der DNA, die Desoxyribose. Reduziertes Glutathion schütz viele Enzyme mit der "SH"-Gruppe und fördert so die Überlebensfähigkeit der Zelle gegen oxidativen Stress wie beispielsweise UV-Strahlung. Aus den genannten Gründen ist G6PDH ein sehr wichtiges Enzym für die Regeneration der Haut, für die Synthese essentieller Substanzen und für den Schutz der Zellen vor oxidativen Stress.

Die Bestimmung der G6PDH-Aktivität erfolgte nach dem von Natsuko Okada und Yukio Kitano in: Arch. Dermatol. Res., 271 (3): 341-346, 1981 beschriebenen Verfahren durch in vitro Bestimmung der enzymatischen Aktivität der Glucose-6-phosphate Dehydrogenase in humanen Fibroblasten.

Der DNA-Gehalt wurde nach der von Desaulniers in Toxic. In vitro 12(4), 409-422 (1998) beschriebenen Methode ermittelt. Die Inkubationszeit der Fibroblasten betrug jeweils 3 Tage und 6 Tage. Die Ergebnisse sind in Tabelle 7 zusammengefaßt. Angegeben ist jeweils das Mittel von 8 Versuchen bei Dreifachbestimmung.

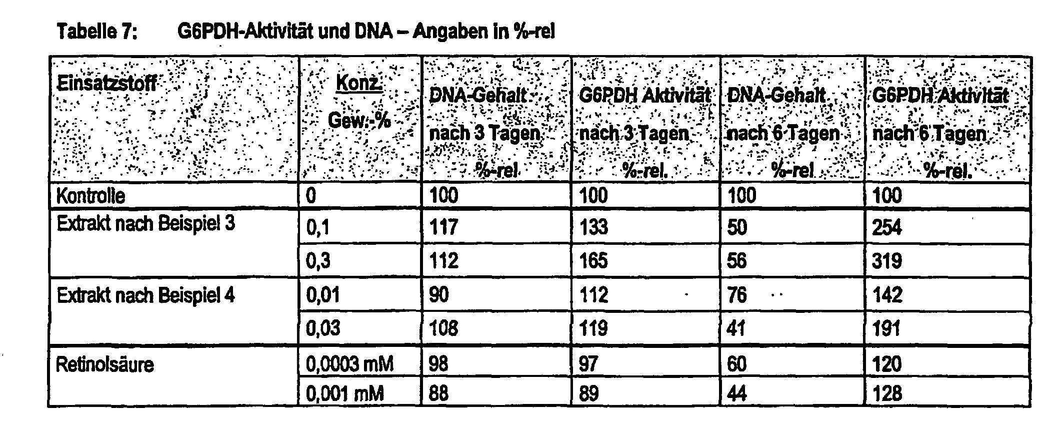

Die Ergebnisse in der Tabelle 7 zeigen, dass die Proteinfraktion aus der Pflanze Argania spinosa mit einer Konzentraten von 0,03 und 0,1 Gew.-% die Aktivität von G6PDH in humanen Fibroblasten nach 6 Tagen extrem erhöht hat. Mit diesen Test kann belegt werden, dass die Proteinfraktionen aus den Rückständen der Ölgewinnung von Argania spinosa ein hohes Potential besitzen menschliche Haut vor Stress wie beispielsweise UV, Umwettverschmutzung zu schützen oder gegen Hautalterung zu wirken.

### 11. Beispielrezepturen kosmetischer Mittel mit nativen Proteinen aus Extrakten der Pflanze Argania spinosa

Die gemäß Beispiel 1 bis 4 erhaltenen Extrakte wurden in den folgenden erfindungsgemäßen Rezepturen K1 bis K21 sowie 1 bis 30 eingesetzt. Die so hergestellten kosmetischen Mittel zeigten gegenüber den Vergleichsrezepturen V1, V2 und V3 sehr gute hautpflegende Eigenschaften bei gleichzeitig guter Hautverträglichkeit. Darüber hinaus sind die erfindungsgemäßen Mittel stabil gegen oxidative Zersetzung.
Alle in der Tabelle 3-6 aufgeführten und verwendeten Substanzen mit registriertem Warenzeichen ® sind Marken und Produkte der COGNIS Gruppe.

**Tabelle 8:**

| **Softcreme Rezepturen K1 bis K7** | | | | | | | | |
|---|---|---|---|---|---|---|---|---|
| (Alle Angaben in Gew.-% bezogen auf das kosmetische Mittel) | | | | | | | | |
| INCI Bezeichnung | K1 | K2 | K3 | K4 | K5 | K6 | K7 | V1 |
| Glyceryl Stearate (and) Ceteareth-12/20 | 8,0 | 8,0 | 8,0 | 8,0 | 8,0 | 8,0 | 8,0 | 8,0 |
| (and) Cetearyl Alcohol (and) Cetyl | | | | | | | | |
| Palmitate | | | | | | | | |
| cetearyl Alcohol | 2,0 | 2,0 | 2,0 | 2,0 | 2,0 | 2,0 | 2,0 | 2,0 |
| Dicaprylyl Ether | 2,0 | 2,0 | 2,0 | 2,0 | 2,0 | 2,0 | 2,0 | 2,0 |
| Cocoglycerides | 3,0 | 3,0 | 3,0 | 3,0 | 3,0 | 3,0 | 3,0 | 3,0 |
| Cetearyl Isononanoate | 3,0 | 3,0 | 3,0 | 3,0 | 3,0 | 3,0 | 3,0 | 3,0 |
| Glycerin (86 Gew.-%ig) | 3,0 | 3,0 | 3,0 | 3,0 | 3,0 | 3,0 | 3,0 | 3,0 |
| Extrakt nach Beispiel 1 bis 4 | 0,5 | 0,5 | 0,5 | 0,5 | 0,5 | 0,5 | 0,5 | - |
| Tocopherol | | 0,5 | | | | | | |
| Allantoin | | | 0,2 | | | | | |
| Bisabolol | | | | 0,5 | | | | |
| Chitosan (Hydagen CMF) | | | | | 10,0 | | | |
| Desoxyribonucleinsäure ¹⁾ | | | | | | 0,5 | | |
| Panthenol | | | | | | | 0,5 | |
| Wasser | | | | Ad 100 | | | | |

**Tabelle 9:**

| **Nachtcremerezepturen K8 bis K14** | | | | | | | | |
|---|---|---|---|---|---|---|---|---|
| (Alle Angaben in Gew.-% bezogen auf das kosmetische Mittel) | | | | | | | | |
| INCI Bezeichnung | K8 | K9 | K10 | K11 | K12 | K13 | K14 | V2 |
| Polyglyceryl-2 Dipolyhydroxystearate | 4,0 | 4,0 | 4,0 | 4,0 | 4,0 | 4,0 | 4,0 | 5,0 |
| Polyglyceryl-3 Diisostearate | 2,0 | 2,0 | 2,0 | 2,0 | 2,0 | 2,0 | 2,0 | 2,0 |
| Cera Alba | 2,0 | 2,0 | 2,0 | 2,0 | 2,0 | 2,0 | 2,0 | 2,0 |
| Zinc Stearate | 2,0 | 2,0 | 2,0 | 2,0 | 2,0 | 2,0 | 2,0 | 2,0 |
| Cocoglycerides | 3,0 | 3,0 | 3,0 | 3,0 | 3,0 | 3,0 | 3,0 | 3,0 |
| Cetaeryl Isononanoate | 8,0 | 8,0 | 8,0 | 8,0 | 8,0 | 8,0 | 8,0 | 8,0 |
| Dicaprylyl Ether | 5,0 | 5,0 | 5,0 | 5,0 | 5,0 | 5,0 | 5,0 | 5,0 |
| Magnesiumsulfate | 1,0 | 1,0 | 1,0 | 1,0 | 1,0 | 1,0 | 1,0 | 1,0 |
| Glycerin (86 Gew.-%ig) | 5,0 | 5,0 | 5,0 | 5,0 | 5,0 | 5,0 | 5,0 | 5,0 |
| Extrakt nach Beispiel 1 bis 4 | 0,5 | 0,5 | 0,5 | 0,5 | 0,5 | 0,5 | 0,5 | - |
| Tocopherol | | 0,5 | | | | | | |
| Allantoin | | | 0,2 | | | | | |
| Bisabolol | | | | 0,5 | | | | |
| Chitosan (Hydagen CMF) | | | | | 10,0 | | | |
| Desoxyribonucleinsäure ¹⁾ | | | | | | 0,5 | | |
| Panthenol | | | | | | | 0,5 | |
| Wasser | | | | Ad 100 | | | | |

**Tabelle 10:**

| **W/O Bodylotion Rezepturen K15 bis K21** | | | | | | | | |
|---|---|---|---|---|---|---|---|---|
| (Alle Angaben in Gew.-% bezogen auf das kosmetische Mittel) | | | | | | | | |
| INCI-Bezeichnung | K15 | K16 | K17 | K18 | K19 | K20 | K21 | V3 |
| PEG-7 Hydrogenated Castor Oil | 7,0 | 7,0 | 7,0 | 7,0 | 7,0 | 7,0 | 7,0 | 7,0 |
| Decyl Oleate | 7,0 | 7,0 | 7,0 | 7,0 | 7,0 | 7,0 | 7,0 | 7,0 |
| Cetearyl Isononanoate | 7,0 | 7;0 | 7,0 | 7,0 | 7,0 | 7,0 | 7,0 | 7,0 |
| Glycerin (86 Gew: %ig) | 5,0 | 5,0 | 5,0 | 5,0 | 5,0 | 5,0 | 5,0 | 5,0 |
| MgSO₄·7H₂O | 1,0 | 1,0 | 1,0 | 1,0 | 1,0 | 1,0 | 1,0 | 1,0 |
| Extrakt nach Beispiel 1 bis 3 | 1,5 | 1,5 | 1,5 | 1,5 | 1,5 | 1,5 | 1,5 | - |
| Tocopherol | | 0,5 | | | | | | |
| Allantoin | | | 0,2 | | | | | |
| Bisabolol | | | | 0,5 | | | | |
| Chitosan (Hydagen CMF) | | | | | 10,0 | | | |
| Desoxyribonucleinsäure ¹⁾ | | | | | | 0,5 | | |
| Panthenol | | | | | | | 0,5 | |
| Wasser | | | | Ad 100 | | | | |

¹⁾ Desoxyribonucleinsäure: Molekuargewicht ca. 70000, Reinheit (bestimmt durch spektro-photometrische Messung der Absorption bei 260 nm sowie 280 nm): mindestens 1,7.

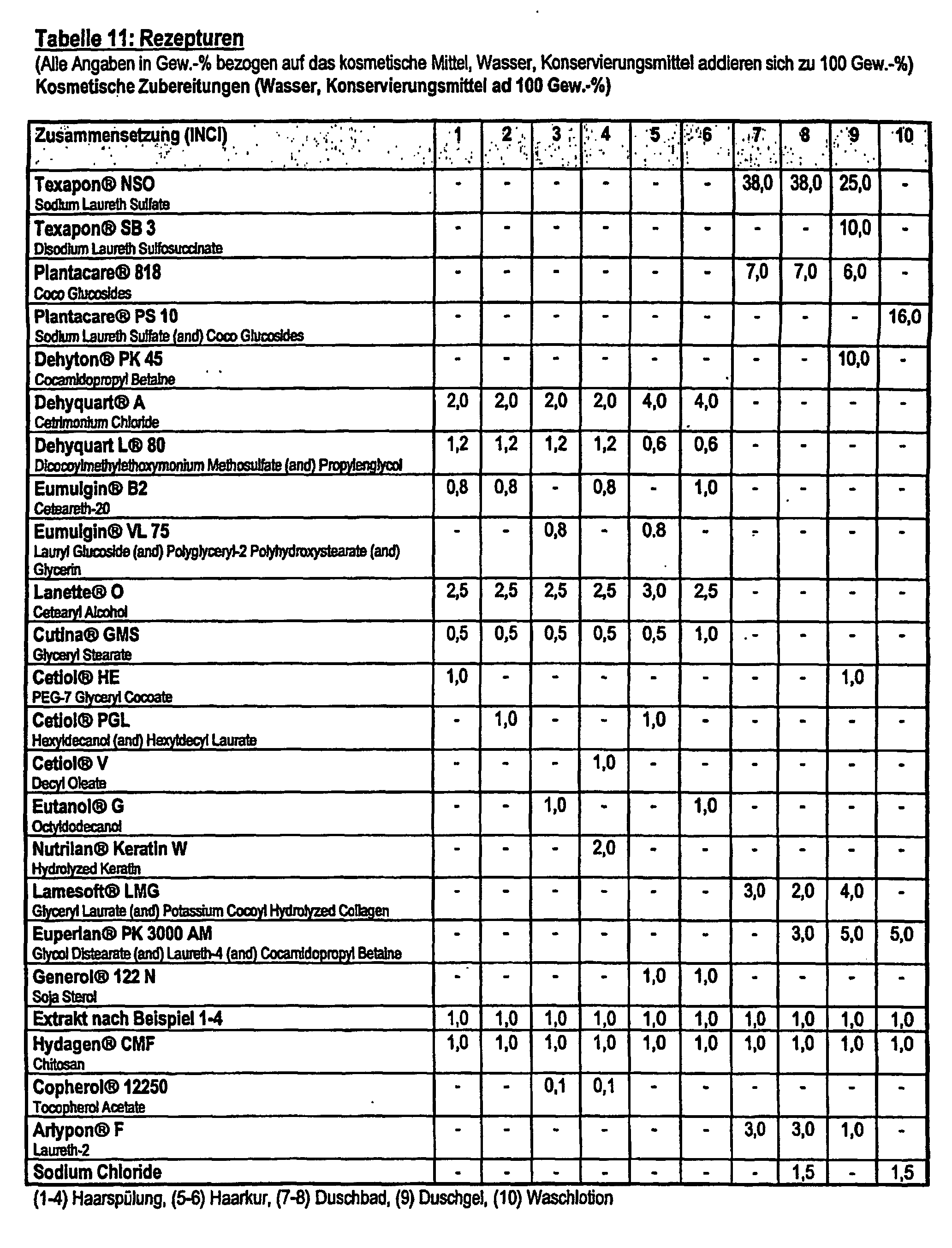

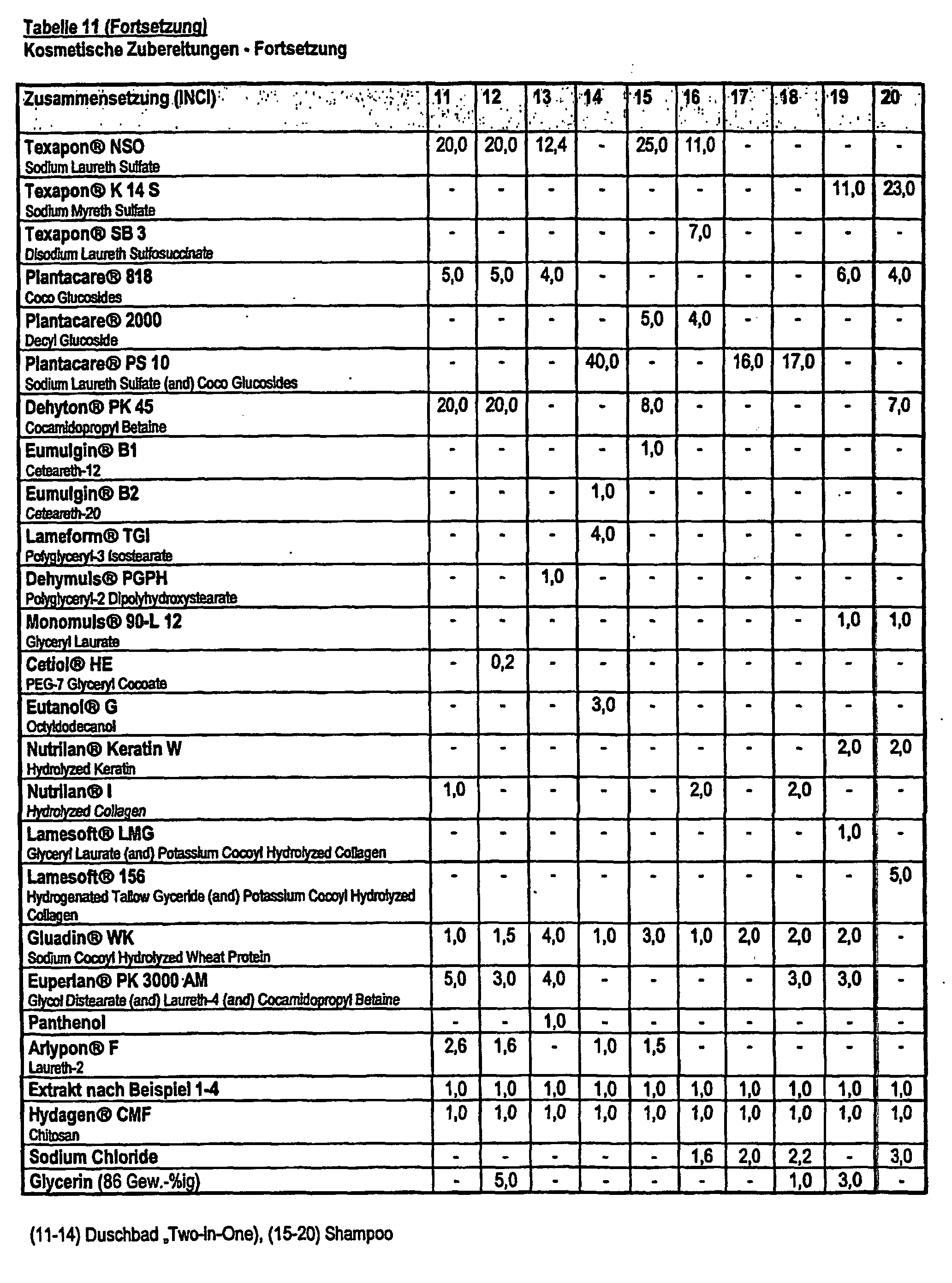

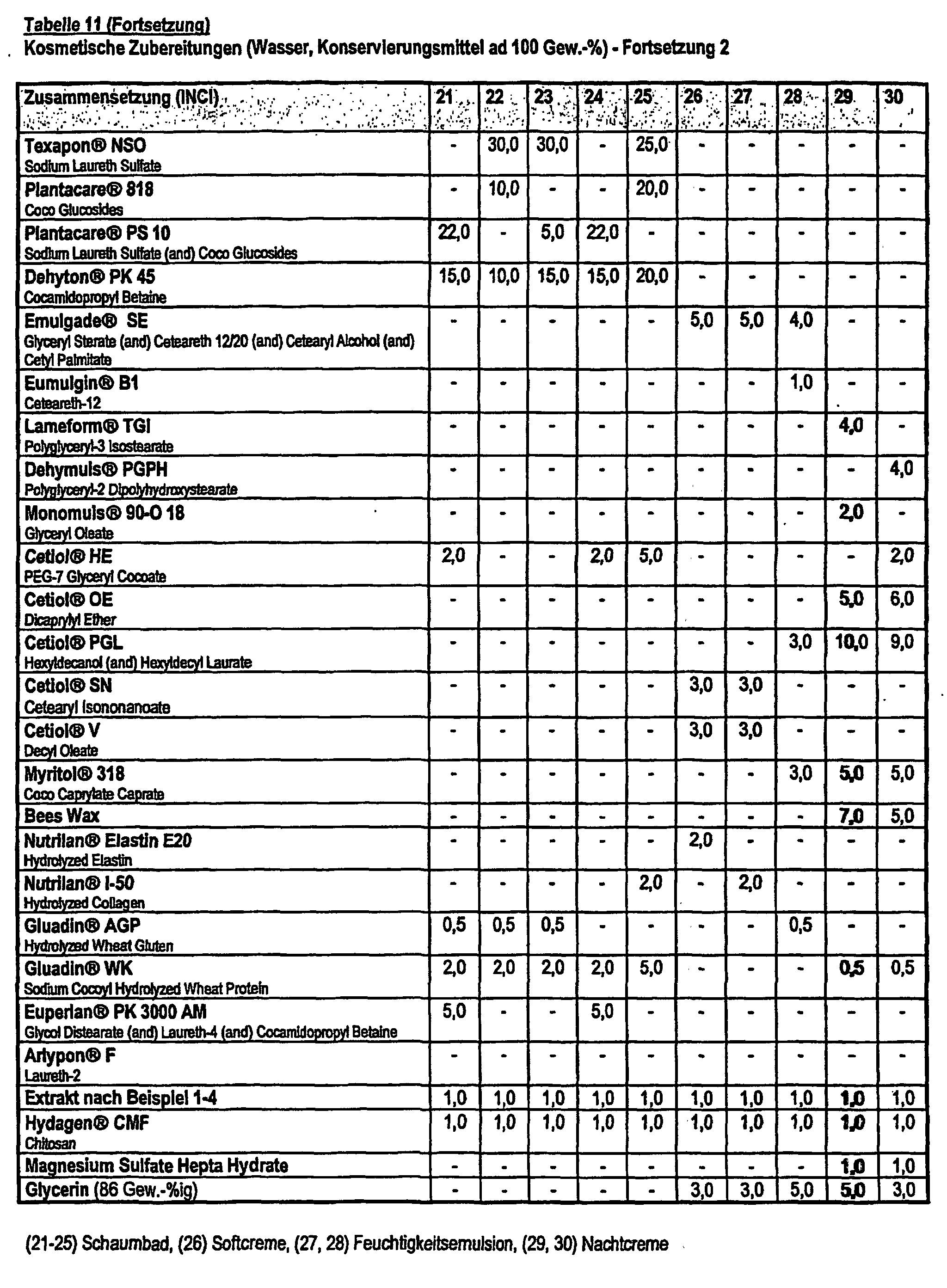

## Patentansprüche

1. Kosmetische und/oder dermopharmazeutische Zubereitungen enthaltend native Proteine aus der Pflanze Argania spinosa als Pflegemittel für Haut und Haare,
wobei die nativen Proteine aus einem Extrakt der Samenkeme von Argania spinosa erhältlich sind, und wobei für die Durchführung der Extraktion Wasser als Lösungsmittel bei einem pH Wert geringer oder gleich 12 verwendet wird.

2. Zubereitungen nach Anspruch 1, **dadurch gekennzeichnet, dass** sie native Proteine enthalten, die erhalten werden aus einem Extrakt der entfetteten Samenkeme von Argania spinosa.

3. Zubereitungen nach Anspruch 1 und/oder 2, **dadurch gekennzeichnet, dass** sie native Proteine enthalten, die erhalten werden durch wässrige Extraktion bei einem pH-Wert geringer oder gleich 12 und gegebenenfalls einer anschließenden Trocknung.

4. Zubereitungen nach einem der Ansprüche 1 bis 3, **dadurch gekennzeichnet, dass** das Molekulargewicht der nativen Proteine größer 500.000 Da ist.

5. Zubereitungen nach einem der Ansprüche 1 bis 3, **dadurch gekennzeichnet, dass** das Molekulargewicht der nativen Proteine im Bereich von 170.000 Da bis 250.000 ist.

6. Zubereitungen nach einem der Ansprüche 1 bis 3, **dadurch gekennzeichnet, dass** das Molekulargewicht der nativen Proteine im Bereich von 10.000 Da bis 18.000 Da ist

7. Zubereitungen nach einem der Ansprüche 1 bis 6, **dadurch gekennzeichnet, dass** sie native Proteine in Form eines Extraktes mit einem Aktivsubstanzgehalt im Bereich von 20 bis 85 Gew.-%-berechnet auf Trockengewicht - enthalten.

8. Zubereitungen nach einem der Ansprüche 1 bis 7, **dadurch gekennzeichnet, dass** sie den native Proteine enthaltenden Extrakt in Mengen von 0,01 bis 25 Gew.-% berechnet bezogen auf die Zubereitung enthalten, mit der Maßgabe, dass sich die Mengenangaben mit Wasser und gegebenenfalls weiteren Hilfs- und Zusatzstoffen zu 100 Gew.-% addieren.

9. Kosmetische Verwendung von nativen Proteinen aus Extrakten der Pflanze Argania spinasa,
wobei die nativen Proteine erhältlich sind wie definiert in Anspruch 1,
als Pflegemittel für Haut und/oder Haare
oder als feuchtigkeitsregulierendes Feuchthaltemittel
oder als Mittel zur Stärkung der Hautbarrierefunktionen
oder als hautstraffendes und hautstabilisierendes Mittel
oder als Sonnenschutzmittel, insbesondere gegen UVA-Strahlung und/oder gegen UVB-Strahlung
oder gegen Hautalterung
oder als revitalisierendes und restrukturierendes Mittel für Haut und/oder Haare.

10. Verwendung von nativen Proteinen aus Extrakten der Pflanze Argania spinosa,
wobei die nativen Proteine erhältlich sind wie definiert in Anspruch 1,
zur Herstellung eines Arzneimittels zur Pflege für Haut und/oder Haare
oder zur Herstellung eines Arzneimittels, das als feuchtigkeitsregulierendes Feuchthaltemittel wirkt,
oder zur Herstellung eines Arzneimittels, das die Hautbarrierefunktionen stärkt,
oder zur Herstellung eines Arzneimittels, das als hautstraffendes und hautstabilisierendes Mittel wirkt,
oder zur Herstellung eines Arzneimittels, das als Sonnenschutzmittel, insbesondere gegen UVA-Strahlung und/oder gegen UVB-Strahlung, wirkt,
oder zur Herstellung eines Arzneimittels gegen Hautalterung
oder zur Herstellung eines Arzneimittels, das als revitalisierendes und restrukturierendes Mittel für Haut und/oder Haare wirkt.

11. Verwendung von nativen Proteinen aus Extrakten der Pflanze Argania spinosa, wobei die nativen Proteine erhältlich sind wie definiert in Anspruch 1, als Wirkstoffe zur Herstellung eines Mittels zur Steigerung der G6PDH-Aktivität im Stoffwechsel.

## Claims

1. Cosmetic and/or dermopharmaceutical preparations containing native proteins from the plant Argania spinosa as a skin- and hair-care component, the native proteins being obtainable from an extract of seed kernels of Argania spinosa and water being used as solvent at or below a pH of 12 for carrying out the extraction process.

2. Preparations as claimed in claim 1, **characterized in that** they contain native proteins obtained from an extract of the defatted seed kernels of Argania spinosa.

3. Preparations as claimed in claim 1 and/or 2, **characterized in that** they contain native proteins obtained by extraction with water at or below a pH of 12, optionally followed by drying.

4. Preparations as claimed in any of claims 1 to 3, **characterized in that** the molecular weight of the native proteins is above 500,000 Da.

5. Preparations as claimed in any of claims 1 to 3, **characterized in that** the molecular weight of the native proteins is in the range from 170,000 Da to 250,000 Da.

6. Preparations as claimed in any of claims 1 to 3, **characterized in that** the molecular weight of the native proteins is in the range from 10,000 to 18,000 Da.

7. Preparations as claimed in any of claims 1 to 6, **characterized in that** they contain native proteins in the form of an extract with an active substance content of 20 to 85% by weight, based on the dry weight.

8. Preparations as claimed in any of claims 1 to 7, **characterized in that** they contain the extract containing the native proteins in quantities of 0.01 to 25% by weight, based on the preparation, with the proviso that these quantities add up to 100% by weight with water and optionally other auxiliaries and additives.

9. Cosmetic use of native proteins from extracts of the plant Argania spinosa, the native proteins being obtainable as defined in claim 1,
as a skin and/or hair care component
or as a moisture-regulating humectant
or as a component for strengthening the skin barrier functions
or as a skin-smoothing and skin-stabilizing component
or as a sun protection component, particularly against UV-A radiation and/or against UV-B radiation
or against ageing of the skin
or as a skin and/or hair revitalizing and restructuring component.

10. Use of native proteins from extracts of the plant Argania spinosa, the native proteins being obtainable as defined in claim 1,
for the production of a skin and/or hair care medicament
or for the production of a medicament acting as a moisture-regulating humectant
or for the production of a medicament which strengthens the skin barrier functions
or for the production of a medicament effective in smoothing and stabilizing the skin
or for the production of a medicament acting as a sun protection component, particularly against UV-A radiation and/or against UV-B radiation
or for the production of a medicament against ageing of the skin
or for the production of a medicament effective in revitalizing and restructuring the skin and/or hair.

11. Use of native proteins from extracts of the plant Argania spinosa, the native proteins being obtainable as defined in claim 1, as active components for the production of a composition for increasing G6PDH activity in the metabolism.

## Revendications

1. Préparations cosmétiques et/ou dermopharmaceutiques contenant des protéines natives provenant de la plante Argania spinosa comme produits de soin pour la peau et les cheveux,
dans lesquelles les protéines natives sont obtenues à partir d'un extrait des noyaux de germes d'Argania spinosa, et pour la réalisation de l'extraction on utilise, comme solvant, de l'eau à un pH inférieur ou égal à 12.

2. Préparations selon la revendication 1,
**caractérisées en ce qu'**
elles contiennent des protéines natives obtenues à partir d'un extrait des noyaux de germes dégraissés d'Argania spinosa.

3. Préparations selon la revendication 1 ou 2,
**caractérisées en ce qu'**
elles contiennent des protéines natives obtenues par extraction aqueuse à un pH inférieur ou égal à 12 et le cas échéant par séchage ultérieur.

4. Préparations selon l'une des revendications là 3,
**caractérisées en ce que**
le poids moléculaire des protéines natives est supérieur à 500 000 daltons.

5. Préparations selon l'une des revendications 1 à 3,
**caractérisées en ce que**
le poids moléculaire des protéines natives se situe dans un intervalle allant de 170 000 à 250 000 daltons.

6. Préparations selon l'une des revendications 1 à 3,
**caractérisées en ce que**
le poids moléculaire des protéines natives se situe dans un intervalle allant de 10 000 à 18 000 daltons.

7. Préparations selon l'une des revendications 1 à 6,
**caractérisées en ce qu'**
elles contiennent des protéines natives sous la forme d'un extrait ayant une teneur en substance active allant de 20 à 85 % en poids - calculée par rapport à la substance sèche.

8. Préparations selon l'une des revendications là 7,
**caractérisées en ce qu'**
elles contiennent l'extrait contenant les protéines natives en quantités de 0,01 à 25 % en poids, calculées par rapport à la préparation, étant précisé que les indications quantitatives se complètent à 100 % en poids avec de l'eau et le cas échéant avec d'autres adjuvants et additifs.

9. Utilisation cosmétique de protéines natives provenant d'extraits de la plante Argania spinosa, les protéines natives pouvant être obtenues comme défini dans la revendication 1,
comme produits de soins pour la peau et/ou les cheveux,
ou comme produits hydratants régulateurs de l'humidité,
ou comme produits pour renforcer les fonctions de barrière de la peau,
ou comme produits de raffermissement et de stabilisation de la peau,
ou comme produits de protection solaire, en particulier contre le rayonnement UVA et/ ou contre le rayonnement UVB,
ou contre le vieillissement de la peau,
ou comme produits revitalisants et restructurants pour la peau et/ou les cheveux.

10. Utilisation de protéines natives provenant d'extraits de la plante Argania spinosa, les protéines natives pouvant être obtenues comme défini dans la revendication 1,
pour la fabrication d'un médicament destiné aux soins de la peau et/ou des cheveux,
ou pour la fabrication d'un médicament qui agit comme produit hydratant régulateur de l'humidité,
ou pour la fabrication d'un médicament qui renforce les fonctions de barrière de la peau,
ou pour la fabrication d'un médicament qui agit comme produit raffermissant et stabilisant pour la peau,
ou pour la fabrication d'un médicament qui agit comme produit de protection solaire, en particulier contre le rayonnement UVA et/ou contre le rayonnement UVB,
ou pour la fabrication d'un médicament contre le vieillissement de la peau,
ou pour la fabrication d'un médicament qui agit comme produit revitalisant et restructurant pour la peau et/ou les cheveux.

11. Utilisation de protéines natives provenant d'extraits de la plante Argania spinosa, les protéines natives étant obtenues comme défini dans la revendication 1, comme substances actives pour la fabrication d'un produit augmentant l'activité de G6PDH dans le métabolisme.
